# EUROPEAN PATENT SPECIFICATION

(11) **EP 3 989 961 B1**
(45) Date of publication and mention of the grant of the patent: **22.11.2023**
(21) Application number: 21705541.7
(22) Date of filing: 18.02.2021
(51) Int. Cl.: A61K 31/197, A61K 31/366, A61K 45/06, A61P 35/00, A61K 31/17, A61K 31/282, A61K 31/495, A61K 31/513

(54) **A PHARMACEUTICAL COMBINATION OF AN ARTEMISININ COMPOUND, 5-AMINOLEVULINIC ACID OR METHYL-5-AMINOLEVULINIC ACID AND A CHEMOTHERAPEUTIC AGENT**
PHARMAZEUTISCHE KOMBINATION AUS EINER ARTEMISININ-VERBINDUNG UND EINEM HÄME-SYNTHESE-MODULATOR ZUR BEHANDLUNG VON KREBS
COMBINAISON PHARMACEUTIQUE D'UN COMPOSÉ DE L'ARTÉMISININE ET D'UN MODULATEUR DE LA SYNTHÈSE DE L'HÈME POUR LE TRAITEMENT DU CANCER

(30) Priority: 19.02.2020 EP 20158341
(43) Date of publication of application: 04.05.2022
(73) Proprietor: JLP Health GmbH, 1130 Wien (AT)
(72) Inventor: ELLING, Ulrich, 2380 Perchtoldsdorf (AT); PENNINGER, Josef, Martin, 1130 Wien (AT)
(74) Representative: Arth, Hans-Lothar
(86) International application number: PCT/EP2021/054039
(87) International publication number: WO 2021/165405

(56) References cited:
- WO-A1-2018/082120
- WO-A1-2019/141988
- CN-A- 108 635 581
- US-A1- 2016 367 674
- TOMOHIRO OSAKI ET AL: "Antimalarial Drugs Enhance the Cytotoxicity of 5-Aminolevulinic Acid-Based Photodynamic Therapy against the Mammary Tumor Cells of Mice In Vitro", MOLECULES, vol. 24, no. 21, 29 October 2019 (2019-10-29), page 3891, XP055716490, DOI: 10.3390/molecules24213891
- TOMOHIRO OSAKI ET AL: "Artesunate Enhances the Cytotoxicity of 5-Aminolevulinic Acid-Based Sonodynamic Therapy against Mouse Mammary Tumor Cells In Vitro", MOLECULES, vol. 22, no. 4, 27 March 2017 (2017-03-27) , page 533, XP055716517, DOI: 10.3390/molecules22040533
- WANG LEI ET AL: "Tumor-targeting core-shell structured nanoparticles for drug procedural controlled release and cancer sonodynamic combined therapy", JOURNAL OF CONTROLLED RELEASE, ELSEVIER, vol. 286, 17 July 2018 (2018-07-17), pages 74-84, XP085477986, ISSN: 0168-3659, DOI: 10.1016/J.JCONREL.2018.07.028
- CAI-PING CHEN ET AL: "Synergistic antitumor activity of artesunate and HDAC inhibitors through elevating heme synthesis via synergistic upregulation of ALAS1 expression", ACTA PHARMACEUTICA SINICA B, vol. 9, no. 5, 1 September 2019 (2019-09-01), pages 937-951, XP055716497, ISSN: 2211-3835, DOI: 10.1016/j.apsb.2019.05.001
- SHIMING ZHANG ET AL: "Heme Mediates Cytotoxicity from Artemisinin and Serves as a General Anti-Proliferation Target", PLOS ONE, vol. 4, no. 10, 28 October 2009 (2009-10-28), page e7472, XP055081754, ISSN: 1932-6203, DOI: 10.1371/journal.pone.0007472
- YUNQIN ZHANG ET AL: "Antitumor Research on Artemisinin and Its Bioactive Derivatives", NATURAL PRODUCTS AND BIOPROSPECTING, vol. 8, no. 4, 9 April 2018 (2018-04-09), pages 303-319, XP055716514, ISSN: 2192-2195, DOI: 10.1007/s13659-018-0162-1
- JIGANG WANG ET AL: "Mechanistic Investigation of the Specific Anticancer Property of Artemisinin and Its Combination with Aminolevulinic Acid for Enhanced Anticolorectal Cancer Activity", ACS CENTRAL SCIENCE, vol. 3, no. 7, 28 June 2017 (2017-06-28), pages 743-750, XP055716694, ISSN: 2374-7943, DOI: 10.1021/acscentsci.7b00156

## Description

### Field of the Invention

The present invention relates to a pharmaceutical composition comprising an artemisinin compound selected from artemisinin, dihydroarteminisin or artesunate or a pharmaceutically acceptable salt, cocrystal, or solvate thereof, and 5-aminolevulinic acid or methyl-5-aminolevulinic acid or a pharmaceutically acceptable salt or solvate thereof and at least one chemotherapeutic agent, as defined in the claims. This pharmaceutical composition is used for the prophylaxis and/or treatment of hematopoietic cancers, brain cancer, pancreatic cancer, liver cancer, breast cancer, and lung cancer such as non-small cell lung cancer. Preferably, the present application provides a pharmaceutical composition comprising artemisinin or dihydroarteminisin or artesunate and 5-aminolevulinic acid or methyl-5-aminolevulinic acid and at least one chemotherapeutic agent, as defined in the claims for use in prophylaxis and/or treatment of brain cancer, in particular glioblastoma.

More preferalbly, the present invention relates to a pharmaceutical composition comprising artemisinin (**1a**), artesunate (**1e**) or dihydroarteminisin (**1b**) and 5-aminolevulinic acid (**2**) and at least one chemotherapeutic agent, selected from the group consisting of temozolomide, lomustine, cisplatin, and 5-fluorouracil, for use in prophylaxis and/or treatment of brain cancer, in particular glioblastoma.

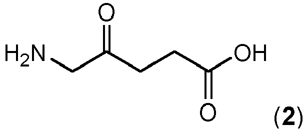

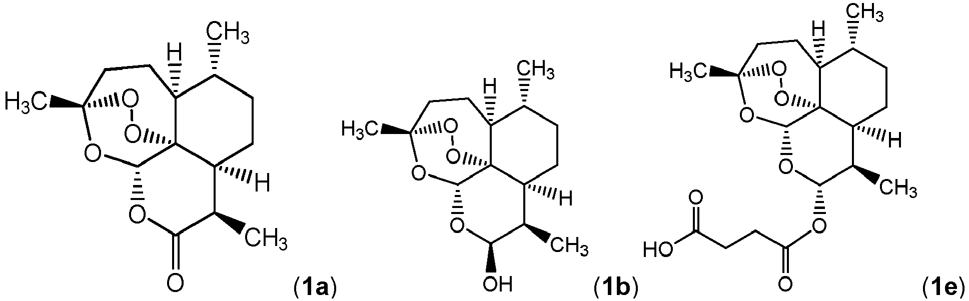

### Background of the Invention

Artemisinin is a natural medical compound that can be isolated from the plant *Artemisia annua,* or sweet wormwood. The substance has been used in traditional Chinese medicine for thousands of years to treat fever, colds and other maladies. In the 1970ies, Artemisinin hast been first described as an antimalarial drug by the Chinese scientist Tu Youyou. Together with its derivatives, Artemisinin is one of the most commonly used antimalarial drugs worldwide. It kills the unicellular parasite P. falciparum, that causes malaria, in all of its life stages {Aweeka, F. T. & German, P. I. Clinical Pharmacology of Artemisinin-Based Combination Therapies. Clinical Pharmacokinetics 47, 91-102 (2008)}. More recently, the potent anti-cancer properties of artemisinin have been recognized. The variety of the different functions of artemisinin and its applications point towards a wide-ranging mechanism of action of the compound in eukaryotic cells.

Artemisinin biochemically is a natural endoperoxide. Its endoperoxide properties are strictly required for its antimalarial effect. Once the endoperoxide bridge is being cleaved, artemisinin gets activated and leads to the production of reactive oxygen species (ROS) and free radicals which subsequently alkylate susceptible proteins and macromolecules in the cell. This alkylation reaction alters the structure and function of proteins, damages the DNA and induces cellular stress, eventually resulting in cell death. Artemisinin associates with a wide range of cellular proteins in a non-specific manner and alters multiple pathways, including glycolysis, protein biosynthesis, mitochondrial processes, and antioxidant responses {Zhang, C.-J. et al. Haem-activated promiscuous targeting of artemisinin in Plasmodium falciparum. Nature Communications 6, 1-11 (2015)} {Ismail, H. M. et al. Artemisinin activity-based probes identify multiple molecular targets within the asexual stage of the malaria parasites Plasmodium falciparum3D7. Proceedings of the National Academy of Sciences of the United States of America 113, 2080-2085 (2016)}, in parallel. While definite regulators or activators of artemisinin are still being debated, free or complexed iron, such as in haem of haemoglobin, are considered the strongest candidates. Artemisinin's promiscuous binding properties, together with the high number of reported functions, have so far hindered the identification of its definite cellular targets {Tilley, L., Straimer, J., Gnädig, N. F., Ralph, S. A. & Fidock, D. A. Artemisinin Action and Resistance in Plasmodium falciparum. Trends in Parasitology 32, 682-696 (2016)}. As an increasing number of artemisinin resistant malaria cases are surfacing, it is imperative to delineate the compounds mechanism of action, in order to expand its applicability as a medical remedy.

5-Aminolevulinic acid (5-ALA) is an intermediate in heme biosynthesis and a tumor marker which is used to mark tumors for surgery. It is a non-protein amino acid.

The specific cytotoxicity of Artemisinin against colorectal cancer (CRC) cells is reported. Jigang Wang *et. al* shows that the artemisinin/aminolevulinic acid combination therapy proves to be more effective than an Artemisinin monotherapy in a xenograft colorectal cancer (CRC) modell (ACS Cent. Sci. 2017, 3, pp. 743-750.)

Glioblastoma, also known as glioblastoma multiforme (GBM), is the most aggressive cancer that begins within the brain. Glioblastomas represent 15% of brain tumors. They can either start from normal brain cells or develop from an existing low-grade astrocytoma. Typically, treatment involves surgery, after which chemotherapy and radiation therapy are used. It is the most common cancer that begins within the brain and the second-most common brain tumor, after meningioma. About three per 100,000 people develop the disease a year. It most often begins around 64 years of age and occurs more commonly in males than females.

Despite maximum treatment, the glioblastoma usually recurs. The typical length of survival following diagnosis is 12 to 15 months, with fewer than 3 to 7% of people surviving longer than five years. Without treatment, survival is typically three months. An effective treatment of glioblastoma is still highly requested.

Therefore, it is the objective of the present invention to provide a pharmaceutical composition useful for prophylaxis and/or treatment of cancer and especially of glioblastoma.

The objective of the present invention is solved by the teaching of the independent claims. Further advantageous features, aspects and details of the invention are evident from the dependent claims, the description, the figures, and the examples of the present application.

Surprisingly, it was found in the present invention that a pharmaceutical composition comprising an artemisinin compound and 5-aminolevulinic acid or methyl-5-aminolevulinic acid and at least one chemotherapeutic agent, as defined in the claims, is useful for prophylaxis and/or treatment of cancer selected from hematopoietic cancers, brain cancer, pancreatic cancer, liver cancer, breast cancer, and lung cancer such as non-small cell lung cancer and especially of glioblastoma.

Said pharmaceutical composition may further comprise a pharmaceutically acceptable carrier, excipient and/or diluent. Optionally, said pharmaceutical composition is used in combination with radiotherapy, immunotherapy, electromagnetic field therapy, and/or hyperthermia therapy.

### Summary of this invention

The present invention provides a pharmaceutical composition comprising
a) an artemisinin compound (**1**)
   or a pharmaceutically acceptable salt, cocrystal, or solvate thereof;
b) 5-aminolevulinic acid (2), methyl-5-aminolevulinic acid (2b), or a pharmaceutically acceptable salt or solvate thereof; and
c) at least one chemotherapeutic agent, preferably one anti-glioblastoma drug.

The artemisinin compound (1) is selected from 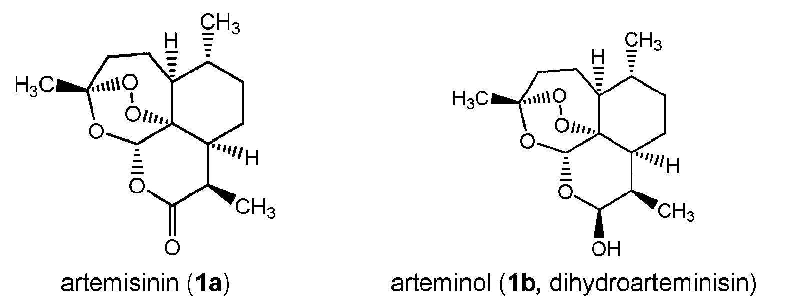 and 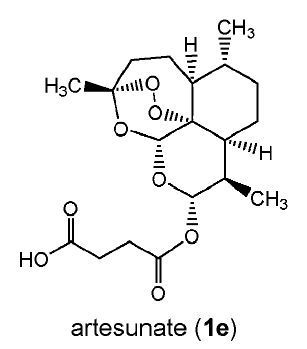 or a pharmaceutically acceptable salt, cocrystal, or solvate of this artemisinin compound (**1**).

The chemotherapeutic agent and preferably the anti-glioblastoma drug is selected from the group consisting of temozolomide, lomustine, cisplatin, 5-fluorouracil.

The pharmaceutical compositions disclosed herein are useful as medicine and especially for the prophylaxis and/or treatment of hematopoietic cancers, brain cancer, pancreatic cancer, liver cancer, breast cancer, and lung cancer such as non-small cell lung cancer and preferably brain cancer.

Preferably, the glioblastoma is selected from subtypes proneural (PN), mesenchymal (MES), and classical (CL) glioblastoma.

For use in prophylaxis and/or treatment of hematopoietic cancers, brain cancer, pancreatic cancer, and liver cancer, breast cancer, and lung cancer such as non-small cell lung cancer, preferably brain cancer, more preferably, glioblastoma, a molar ratio of the artemisinin compound and the 5-aminolevulinic acid or the methyl-5-aminolevulinic acid is preferably in a range of 1:5 to 1:5000, more preferably 1:5 to 1:1000, still more preferably 1:10 to 1:500 in the pharmaceutical composition.

Optionally, for use in prophylaxis and/or treatment of hematopoietic cancers, brain cancer, pancreatic cancer, liver cancer, breast cancer, and lung cancer such as non-small cell lung cancer, preferably brain cancer, more preferably, glioblastoma, the pharmaceutical composition further comprises pharmaceutically acceptable carrier, excipient and/or diluent.

In some embodiments, the pharmaceutical composition is used for prophylaxis and/or treatment of hematopoietic cancers, brain cancer, pancreatic cancer, liver cancer, breast cancer, and lung cancer such as non-small cell lung cancer, preferably brain cancer, more preferably, glioblastoma preferably in combination with a radiotherapy, immunotherapy, electromagnetic field therapy, hyperthermia therapy, chemotherapy, cancer immuntherapy and/or any other small molecule based therapy.

In some embodiments, the pharmaceutical composition is used in a form of tablet, capsule, syrup, solution, suspension, emulsion, or gel.

In some embodiments, for prophylaxis and/or treatment of hematopoietic cancers, brain cancer, pancreatic cancer, liver cancer, breast cancer, and lung cancer such as non-small cell lung cancer, preferably brain cancer, more preferably, glioblastoma, the pharmaceutical composition is administered by oral or parenteral application. Preferably, the parenteral application includes intradermal, intragastral, intracutaneous, intravasal, intravenous, intramuscular, subcutaneous, sublingual, topical, and transdermal application.

In some embodiments, for prophylaxis and/or treatment of hematopoietic cancers, brain cancer, pancreatic cancer, and liver cancer and lung cancer such as non-small cell lung cancer, preferably brain cancer, more preferably glioblastoma, the artemisinin contained in the pharmaceutical composition is administered in a range of 0.01 to 100 mg/kg per body weight per day and the 5-aminolevulinic acid is administered in a range of 0.01 to 200 mg/kg per body weight per day.

In some embodiments, the pharmaceutical composition is used for prophylaxis and/or treatment of glioblastoma preferably in combination with the at least one chemotherapeutic agent, preferably one anti-glioblastoma drug, wherein the at least one chemotherapeutic agent, preferably the at least one anti-glioblastoma drug is administered in a range of 0.01 to 100 mg/kg per body weight per day.

### Description of the invention

Note that the references to methods of treatment in this description are to be interpreted as references to the compounds, pharmaceutical compositions and medicaments of the present invention for use in a method for treatment of the human (or animal) body by therapy.

The present invention provides a pharmaceutical composition comprising
a) an artemisinin compound (**1**)
   or a pharmaceutically acceptable salt, cocrystal, or solvate thereof; and
b) 5-aminolevulinic acid (**2**), methyl-5-aminolevulinic acid (**2b**), or a pharmaceutically acceptable salt or solvate thereof; and
c) at least one chemotherapeutic agent, preferably one anti-glioblastoma drug.

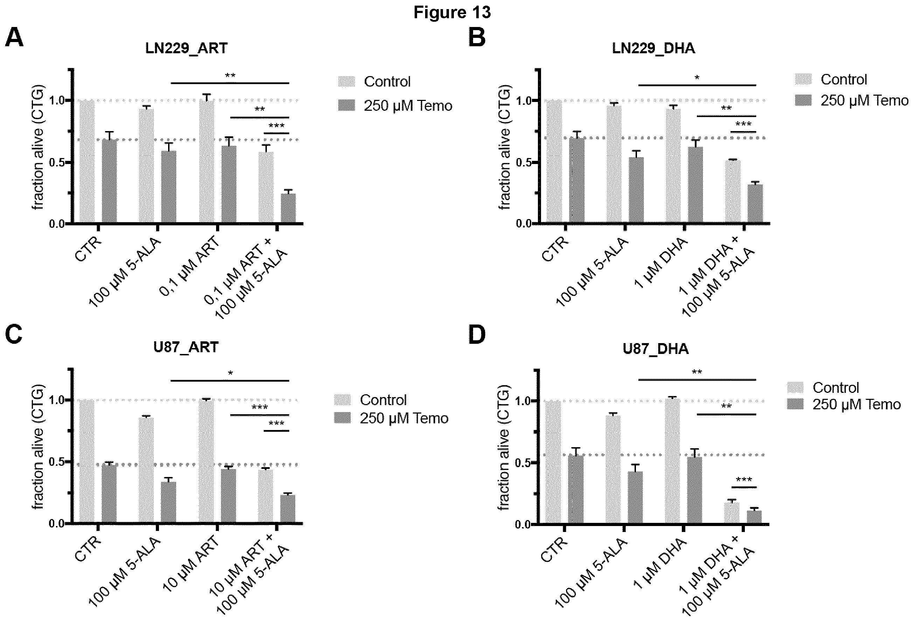

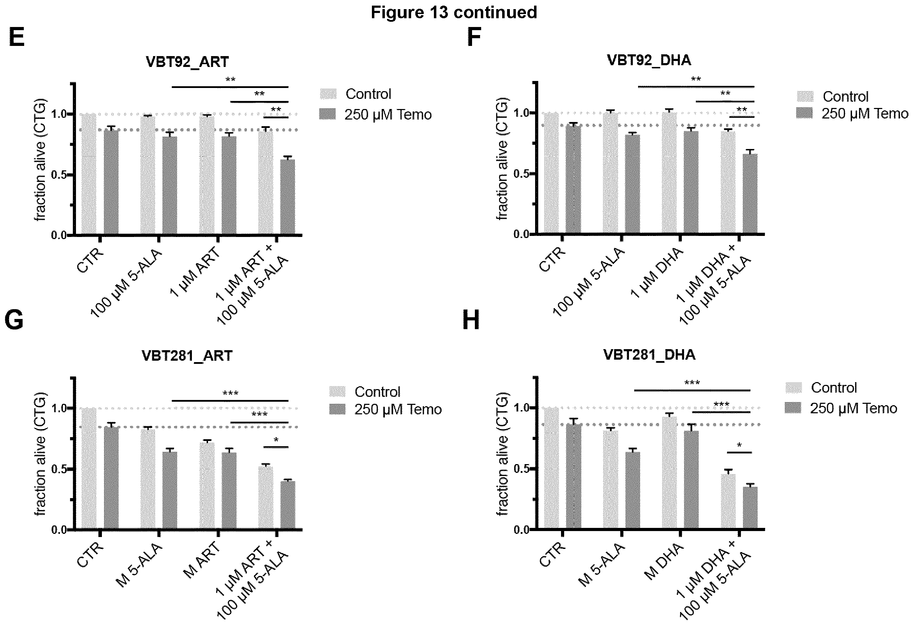

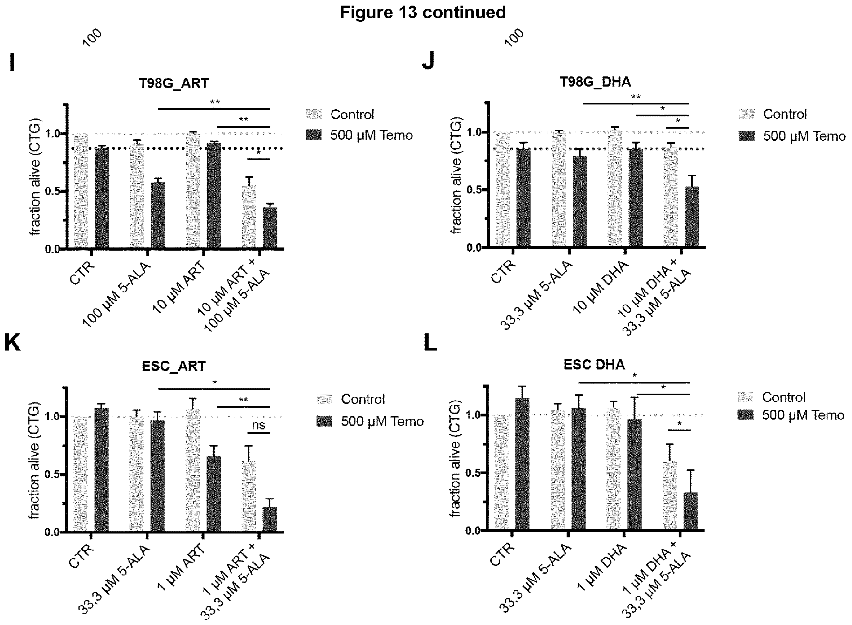

It was surprisingly found that the pharmaceutical compositions disclosed herein containing three pharmaceutically active ingredients, namely an artemisinin compound (**1**) and 5-aminolevulinic acid (**2**) or methyl-5-aminolevulinic acid (**2b**) and at least one chemotherapeutic agent, preferably the at least one anti-glioblastoma drug are considerably more effective than the combinations of only two of these active ingredients as evident from Figures 13 to 19.

The term >>artemisinin compound<< is used herein refers to artemisinin (**1a**), dihydroarteminisin (**1b**), and artesunate (**1e**) as shown below and pharmaceutically acceptable salts, cocrystals, or solvates of the artemisinin compounds (**1**). 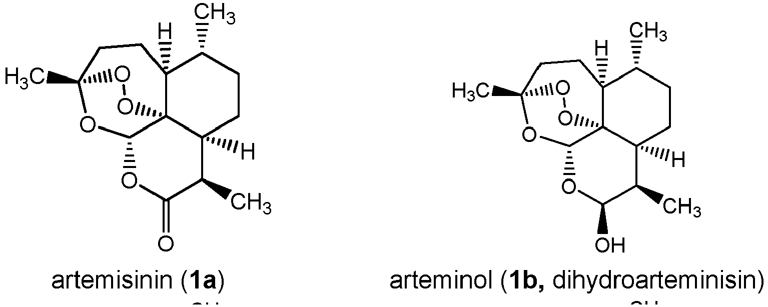 and

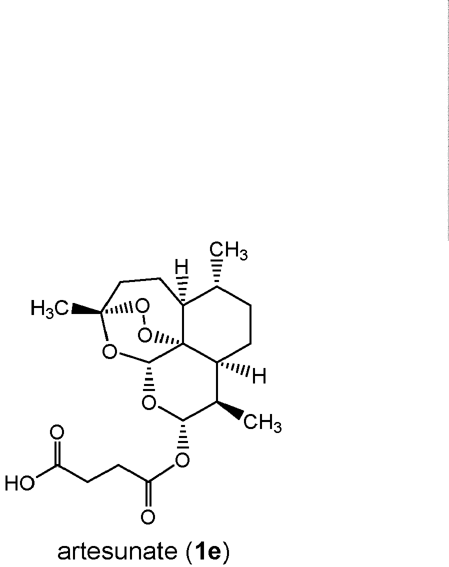

In all pharmaceutical compositions disclosed herein artemisinin (**1a**), dihydroarteminisin (**1b**), and artesunate (**1e**) are preferred as artemisinin compound (**1**). More preferred are artemisinin (**1a**) and artesunate (**1e**) and most preferred is artemisinin (**1a**).

Thus, the present application relates preferably to a pharmaceutical composition comprising
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one chemotherapeutic agent, as defined in the claims.

More preferably the present application relates to a pharmaceutical composition comprising
a) artemisinin (**1a**) or artesunate (**1e**),
   or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one chemotherapeutic agent, as defined in the claims.

Still more preferably the present application relates to a pharmaceutical composition comprising
a) artesunate (**1e**) or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one chemotherapeutic agent, as defined in the claims.

Still more preferably the present application relates to a pharmaceutical composition comprising
a) artemisinin (**1a**) or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one chemotherapeutic agent, as defined in the claims.

Concerning the component b), 5-aminolevulinic acid (**2**) is preferred over methyl-5-aminolevulinic acid (**2b**).

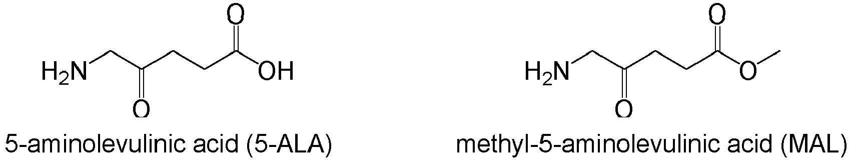

Consequently, present invention provides a pharmaceutical composition comprising
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof; and
b) 5-aminolevulinic acid (**2**) or a pharmaceutically acceptable salt or solvate thereof; and
c) at least one chemotherapeutic agent, as defined in the claims.

More preferably the present application relates to a pharmaceutical composition comprising
a) artemisinin (**1a**) or artesunate (**1e**),
   or a pharmaceutically acceptable salt, cocrystal, or solvate thereof; and
b) 5-aminolevulinic acid (**2**) or a pharmaceutically acceptable salt or solvate thereof; and
c) at least one chemotherapeutic agent, as defined in the claims.

Still more preferably the present application relates to a pharmaceutical composition comprising
a) artesunate (**1e**) or a pharmaceutically acceptable salt, cocrystal, or solvate thereof; and
b) 5-aminolevulinic acid (**2**) or a pharmaceutically acceptable salt or solvate thereof; and
c) at least one chemotherapeutic agent, as defined in the claims.

Still more preferably the present application relates to a pharmaceutical composition comprising
a) artemisinin (**1a**) or a pharmaceutically acceptable salt, cocrystal, or solvate thereof; and
b) 5-aminolevulinic acid (**2**) or a pharmaceutically acceptable salt or solvate thereof; and
c) at least one chemotherapeutic agent, as defined in the claims.

The chemotherapeutic agent, preferably the anti-glioblastoma drug is selected from the group consisting of temozolomide, lomustine, cisplatin, and 5-fluorouracil.

The anit-glioblastoma drug is selected from the group consisting of temozolomide, lomustine, cisplatin, and 5-fluorouracil. Most preferred is temozolomide.

Preliminary data (not claimed) have indicated that the combination of artemisinin and 5-aminolevulinic acid and triptolide or homoharringtonin or dactinomycin or doxorubicin or epirubicin or vinorebine is comparable to the combination of artemisinin and 5-aminolevulinic acid and temozolomide.

Furthermore, the present invention relates to a pharmaceutical composition comprising
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one anti-glioblastoma drug selected from the group consisting of temozolomide, lomustine. Most preferred is temozolomide.

More preferably the present application relates to a pharmaceutical composition comprising
a) artemisinin (**1a**) or artesunate (**1e**),
   or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one anti-glioblastoma drug selected from the group consisting of temozolomide, lomustine, cisplatin, and 5-fluorouracil. Most preferred is temozolomide.

Still more preferably the present application relates to a pharmaceutical composition comprising
a) artesunate (**1e**) or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one anti-glioblastoma drug selected from the group consisting of temozolomide, lomustine, cisplatin, and 5-fluorouracil. Most preferred is temozolomide.

Still more preferably the present application relates to a pharmaceutical composition comprising
a) artemisinin (**1a**) or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one anti-glioblastoma drug selected from the group consisting of temozolomide, lomustine, cisplatin, and 5-fluorouracil. Most preferred is temozolomide.

Furthermore, the present application relates to a pharmaceutical composition comprising
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof;
b) 5-aminolevulinic acid (**2**) or a pharmaceutically acceptable salt or solvate thereof; and
c) at least one anti-glioblastoma drug selected from the group consisting of temozolomide, lomustine, cisplatin, and 5-fluorouracil. Most preferred is temozolomide.

More preferably the present application relates to a pharmaceutical composition comprising
a) artemisinin (**1a**) or artesunate (**1e**),
   or a pharmaceutically acceptable salt, cocrystal, or solvate thereof;
b) 5-aminolevulinic acid (**2**) or a pharmaceutically acceptable salt or solvate thereof; and
c) at least one anti-glioblastoma drug selected from the group consisting of temozolomide, lomustine, cisplatin, and 5-fluorouracil. Most preferred is temozolomide.

Still more preferably the present application relates to a pharmaceutical composition comprising
a) artesunate (**1e**) or a pharmaceutically acceptable salt, cocrystal, or solvate thereof;
b) 5-aminolevulinic acid (**2**) or a pharmaceutically acceptable salt or solvate thereof; and
c) at least one anti-glioblastoma drug selected from the group consisting of temozolomide, lomustine, cisplatin, and 5-fluorouracil. Most preferred is temozolomide.

Still more preferably the present application relates to a pharmaceutical composition comprising
a) artemisinin (**1a**) or a pharmaceutically acceptable salt, cocrystal, or solvate thereof;
b) 5-aminolevulinic acid (**2**) or a pharmaceutically acceptable salt or solvate thereof; and
c) at least one anti-glioblastoma drug selected from the group consisting of temozolomide, lomustine, cisplatin, and 5-fluorouracil. Most preferred is temozolomide.

As used herein, the terms **"artemisinin compound"** means a compound selected from the group comprising or consisting of artemisinin (ART or Art or **1a**),
artesunate (ARS or **1e**), and dihydroarteminisinin (arteminol or DHA or **1b**).

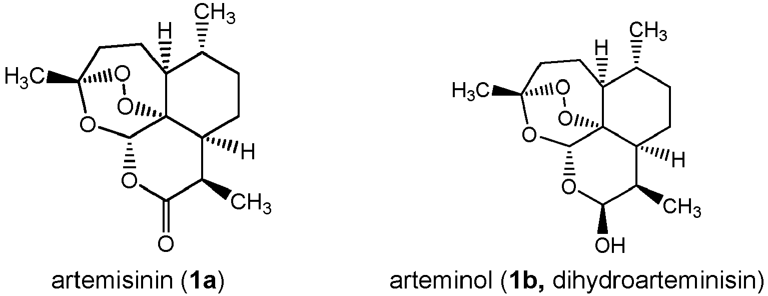

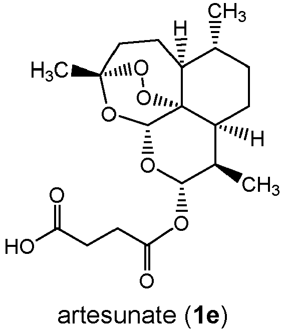

The active metabolite of the artemisinin compound (**1**) in general is dihydroarteminisin (DHA). As used herein, the terms "artemisinin", "ART", "Art" are used interchangeably and encompass a well known compound having the chemical structure (**1a**).

As used herein, and unless otherwise specified, the term "pharmaceutically acceptable salt(s) of an artemisinin compound (**1**)", includes, but is not limited to, salts of acidic or basic moieties of compounds described herein. Basic moieties are capable of forming a wide variety of salts with various inorganic and organic acids. The acids that can be used to prepare pharmaceutically acceptable acid addition salts of such basic compounds are those that form non-toxic acid addition salts, e.g ., salts containing pharmacologically acceptable anions. Suitable organic acids include, but are not limited to, maleic, fumaric, benzoic, ascorbic, succinic, acetic, formic, oxalic, propionic, tartaric, salicylic, citric, gluconic, lactic, mandelic, cinnamic, oleic, tannic, aspartic, stearic, palmitic, glycolic, glutamic, gluconic, glucaronic, saccharic, isonicotinic, methanesulfonic, ethanesulfonic, p-toluenesulfonic, benzenesulfonic acids, or pamoic (e.g, 1,1'-methylene-bis-(2-hydroxy-3-naphthoate)) acids. Suitable inorganic acids include, but are not limited to, hydrochloric, hydrobromic, hydroiodic, sulfuric, phosphoric, or nitric acids. Compounds that include an amine moiety can form pharmaceutically acceptable salts with various amino acids, in addition to the acids mentioned above. Chemical moieties that are acidic in nature are capable of forming base salts with various pharmacologically acceptable cations. Examples of such salts are alkali metal or alkaline earth metal salts and, particularly, calcium, magnesium, sodium, lithium, zinc, potassium, or iron salts.

As used herein, and unless otherwise specified, the term "pharmaceutically acceptable salt(s) of 5-aminolevulinic acid (**2**)" or "pharmaceutically acceptable salt(s) of methyl-5-aminolevulinic acid (**2b**)", includes, but is not limited to, salts of acidic or basic moieties of compounds described herein. Basic moieties are capable of forming a wide variety of salts with various inorganic and organic acids. The acids that can be used to prepare pharmaceutically acceptable acid addition salts of such basic compounds are those that form non-toxic acid addition salts, e.g ., salts containing pharmacologically acceptable anions. Suitable organic acids include, but are not limited to, maleic, fumaric, benzoic, ascorbic, succinic, acetic, formic, oxalic, propionic, tartaric, salicylic, citric, gluconic, lactic, mandelic, cinnamic, oleic, tannic, aspartic, stearic, palmitic, glycolic, glutamic, gluconic, glucaronic, saccharic, isonicotinic, methanesulfonic, ethanesulfonic, p-toluenesulfonic, benzenesulfonic acids, or pamoic (e.g, 1,1'-methylene-bis-(2-hydroxy-3-naphthoate)) acids. Suitable inorganic acids include, but are not limited to, hydrochloric, hydrobromic, hydroiodic, sulfuric, phosphoric, or nitric acids. Compounds that include an amine moiety can form pharmaceutically acceptable salts with various amino acids, in addition to the acids mentioned above. Chemical moieties that are acidic in nature are capable of forming base salts with various pharmacologically acceptable cations. Examples of such salts are alkali metal or alkaline earth metal salts and, particularly, calcium, magnesium, sodium, lithium, zinc, potassium, or iron salts.

As used herein, and unless otherwise specified, the term "cocrystals", "pharmaceutically acceptable cocrystal(s) of artemisinin compound", are crystalline materials composed of two or more different molecules, i.e. artemisin compound as active pharmaceutical ingredient (API) and cocrystal formers ("coformers"), in the same crystal lattice.

Co-crystal is a formation of mainly hydrogen bond between the drug molecule and coformer so API regardless of acidic, basic, or ionisable groups could potentially be cocrystallized. Cocrystallization can improve physiochemical properties like solubility, dissolution rate, chemical stability and melting point. Interactions which are responsible for the formation of co-crystals include hydrogen bonding, π-stacking, and Van der Waals forces.

The preferred coformer of cocrystal of artemisinin compound may include, but not be limited to nicotinamide, ascorbic acid, urea, tromethamine, theophylline, theophylline-7-acetic acid, theobromine, sulfamide, sucrose, sorbitol, saccharin, pyridoxine, phloroglucinol, paracetamol, N-methylglucosamine, methanesulfonic acid, D-mannitol, malonic acid.

As used herein, and unless otherwise specified, the term"solvate" means a compound that further includes a stoichiometric or non-stoichiometric amount of solvent bound by non-covalent intermolecular forces. The solvent may include, but not be limited to, alcohols such as ethanol, propanol, isopropanol, n-butanol, glycol, N-methyl-2-pyrrolidone, acetonitril, N,N'-dimethylformamide, dimethylsulfoxide, water. Where the solvent is water, the solvate is a hydrate.

As used herein, the terms "5-aminolevulinic acid", "delta-aminolevulinic acid", "ALA" and "5-ALA" are used interchangeably and encompass 5-amino-4-oxopentanoic acid of the chemical structure (**2**).

In humans, 5-aminolevulinic acid is a precursor to heme. 5-aminolevulinic acid goes through a series of transformations in the cytosol and is finally converted to Protoporphyrin IX inside the mitochondria. This protoporphyrin molecule chelates with iron in presence of enzyme ferrochelatase to produce Heme. Administration of 5-aminolevulinic acid can thus be used to increase intracellular heme levels and to increase intracellular iron concentrations. 5-Aminolevulinic acid can be administered as such or in any pharmaceutically acceptable physical form. For example, 5-aminolevulinic acid can be in the form of a pharmaceutically acceptable salt or solvate.

As used herein, the term **"pharmaceutical salts of 5-aminolevulinic acid**" will typically be derived from ALA or an ALA derivative and a mono-protic acid, e.g. a sulfonic acid such as methanesulfonic acid, thereby forming a 1:1 salt. Alternatively, salts may be formed between ALA or an ALA derivative and a di- or tri-protic acid, e.g. a sulfonic acid such as ethane-1, 2-disulfonic acid, sulfuric acid or phosphoric acid. In the case where an acid having more than one acidic proton is used, the resulting compound may have a stoichiometric ratio other than 1:1, for example 2:1 (ALA:acid) or 3:1 (ALA:acid), or may comprise a mixture of salts having varying levels of stoichiometry. In the case of sulfuric acid, for example, a 2:1 (ALA:acid) salt may form whereas in the case of phosphoric acid a 3:1 (ALA:acid) salt may form. Polyprotic acids are also capable of forming other salts with ALA or an ALA derivative. Sulfuric acid, for instance, may provide a 1:1 (ALA: acid) salt based on the HSO₄⁻ anion and phosphoric acid may provide both a 2:1 (ALA: acid) and 1:1 (ALA: acid) salt (or combination thereof) based on the HPO₄²⁻ and H₂PO₄⁻ anions, respectively. Moreover, polyprotic acids can also form other salts, e.g. 1:1 salts, with ALA or its derivatives (e.g. with ALA esters) in the form of salts with other physiologically acceptable bases, such as sodium hydroxide, calcium hydroxide, potassium hydroxide and meglumine. Salts according to the invention preferably derive from an acid having a pKa of about 4 or less, more preferably about 3 or less. The acid may be inorganic or organic. Preferred inorganic acids include hydrobromic acid, hydrochloric acid, sulfuric acid, phosphoric acid and nitric acid. Preferred organic acids include sulfonic acid and sulfonic acid derivatives. Those salts derived from hydrochloric acid, nitric acid, sulfonic acid and sulfonic acid derivatives are especially preferred. The term "sulfonic acid" is intended to include any organic compound containing at least one -SO₃H group. Preferably, this may comprise 1, 2 or 3 -SO₃H groups, most preferably 1 or 2, e.g. 1. When used in relation to sulfonic acid, the term "derivatives" is intended to encompass any such compounds containing at least one (preferably 1, 2 or 3 , most preferably 1 or 2, e.g. 1) -SO₃X group (where X is a physiologically tolerable cation, such as a sodium, calcium, potassium, magnesium or meglumine cation).

As used herein, the term **"treating"** or **"treatment"** encompasses to reversing, alleviating or inhibiting the progress of a disease, disorder or condition, or improvement of one or more symptoms of such disease, disorder or condition, to which such term applies. As used herein, "treating" or "treatment" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein the term "treating" may also refer to preventing the recurrence of a disease, disorder or condition or of one or more symptoms associated with such disease, disorder or condition. The terms "treatment" and "therapeutically," as used herein, refer to the act of treating, as "treating" is defined above.

Preferably, a disease, disorder is a cancer selected from hematopoietic cancers, brain cancer, pancreatic cancer, liver cancer, breast cancer, and lung cancer such as non-small cell lung cancer, preferably barin cancer, in particular glioblastoma.

The term **"effective amount"** as used herein, refers to the amount of a required to a cancer selected from hematopoietic cancers, brain cancer, pancreatic cancer, liver cancer, breast cancer, and lung cancer such as non-small cell lung cancer, preferably brain cancer, in particular glioblastoma relative to an untreated patient. The effective amount of active compound(s) used to practice the present invention for therapeutic treatment of a disease varies depending upon the manner of administration, the age, body weight, and general health of the subject. Ultimately, the attending physician or veterinarian will decide the appropriate amount and dosage regimen. Such amount is referred to as an "effective" amount.

The term **"patient"** or **"subject,"** as used herein, refers to a mammalian subject (primates (e.g. humans, cows, sheep, goats, pigs, horses, dogs, cats, rabbits, rats, mice and the like), preferably a human subject, that has, is suspected of having, or is or may be susceptible to a condition associated with a cancer selected from hematopoietic cancers, brain cancer, pancreatic cancer, liver cancer, breast cancer, and lung cancer such as non-small cell lung cancer, prefearlby brain cancer, in particular glioblastoma. In one embodiment, the present method may be used for treating a patient who suffers from multiple sclerosis, e.g. with any symptoms as discussed above. In another embodiment, the present method may also be used to prevent a perspective patient from getting glioblastoma.

Glioblastoma, also known as glioblastoma multiforme (GBM), is the most aggressive cancer that begins within the brain. The glioblastoma has the subtypes proneural (PN), mesenchymal (MES), and classical (CL) glioblastoma. Therefore, in one embodiment, the glioblastoma is preferably selected from subtypes proneural (PN), mesenchymal (MES), and classical (CL) glioblastoma.

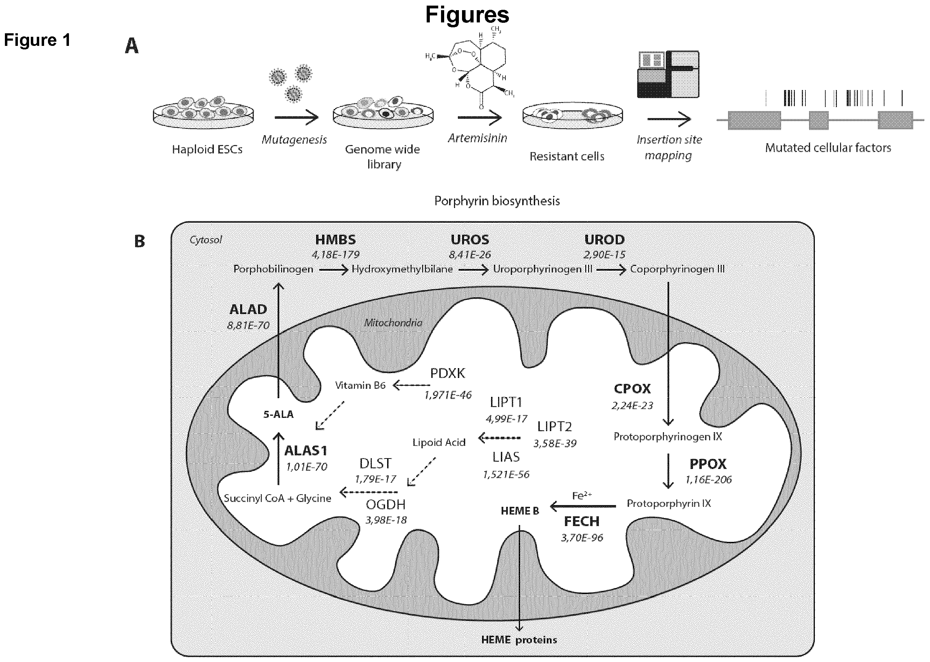

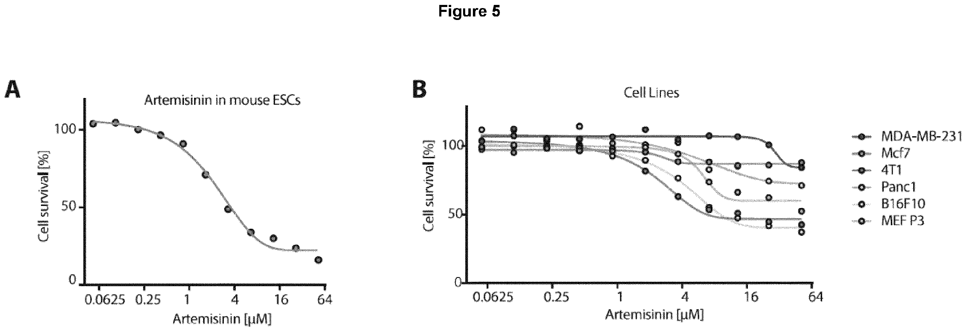

The applicant conducted a screen to identify targets of artemisinin in haploid murine stem cells (haESC) using genome-wide mutagenesis [Figure 1A]. In order to establish screening conditions, the applicant determined LD-values for the compound in wildtype haESCs and assessed growth rates in different murine tumor cell lines [Figure 5A, 5B]. For the generation of a genome-wide mutant ESC libraries, the applicant used two targeting systems for insertional mutagenesis: Retrovirus and Tol2 transposons { Schnütgen, F. et al. Enhanced gene trapping in mouse embryonic stem cells. Nucleic Acids Research 36, e133-e133 (2008)}. Selection of those independent libraries led to the recovery of Artemisinin resistant colonies from the mutagenized cell pools, but not from control cells. Upon expansion and mapping of their insertion sites, enrichment scores based on loss-of-function (LOF) analysis were determined.

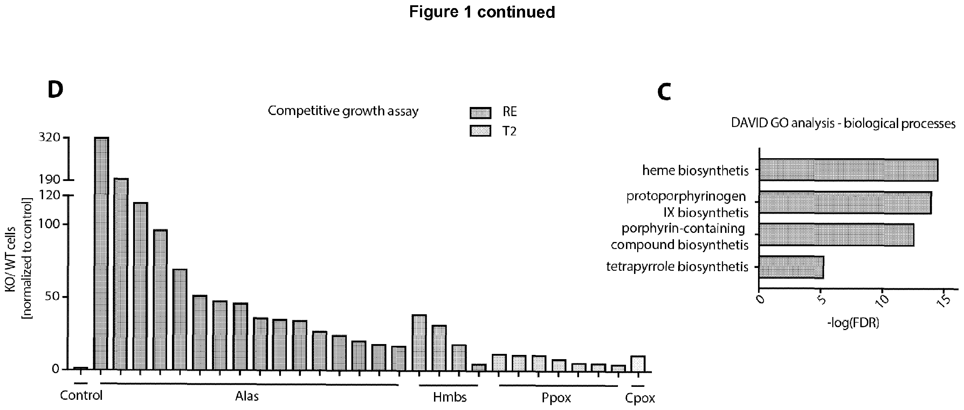

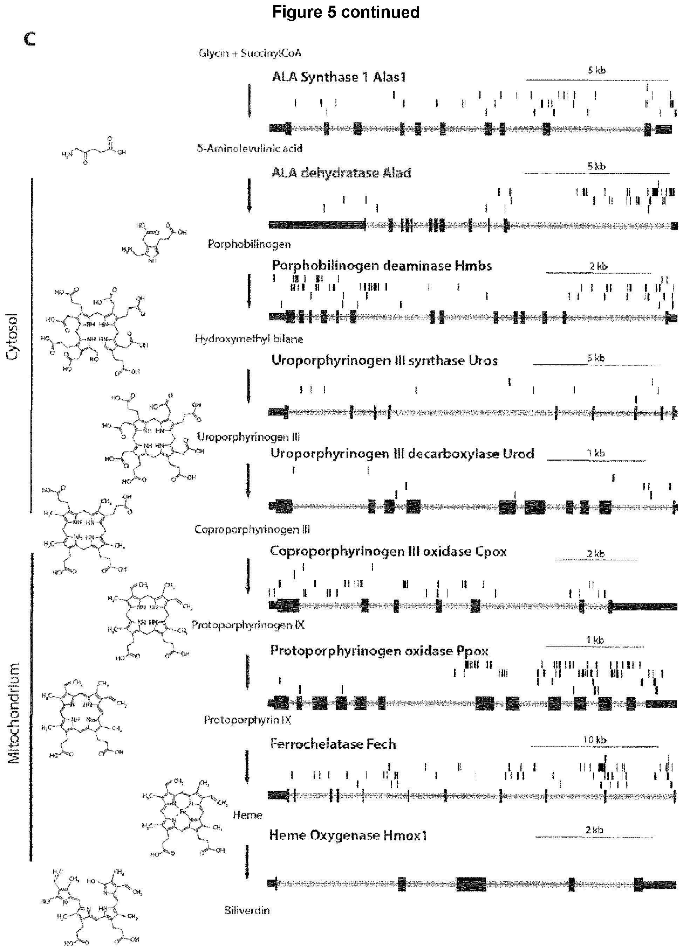

Haploid mutagenesis and artemisinin compound screening identified the enzyme, protoporphyrinogen oxidase, PPOX, as the top hit in both mutant libraries. PPOX is an inner mitochondrial membrane enzyme involved in cellular porphyrin or heme production. Intriguingly, genetic mutants of every single enzyme of the porphyrin biosynthesis pathway, both in mitochondria (Alas1, Cpox, Ppox, Fech) and the cytoplasm (Alad, Hmbs, Uros, Urod) were recovered from the screen, as well as essentially all relevant co-factor-producing enzymes (Lias, Ogdh, Dlst, Lip1, Lipt2, Pdxk) as well as pathways feeding into the protoporphyrin or heme biosynthesis [Figure 1B; Figure 5C]. GO term analysis of the artemisinin screening profile in ESCs confirmed porphyrin biosynthesis as the most significantly targeted pathway in the context of artemisinin toxicity [Figure 1C]. Thus, forward genetic screening in haploid ESCs, delineates porphyrinbiosynthesis as the definivie and essential pathway for artemisinin cytotoxicity.

To confirm the hits from the high-throughput artemisinin screen on the individual level, the applicant tested single mutant ESC clones for their susceptibility to artemisinin. Individual colonies were recovered from both mutant libraries directly from the screen and used to establish single cell lines. From these, the applicant generated labelled wild-type (mCherry_ Cre) and knockout (GFP) sister cell clones (via Cre-recombinase-mediated reversion of the knock-out cassette) and monitored their relative growth rates each in presence of artemisinin. While ratios of mixed labelled cells were constant in absence of the compound or control cells, artemisinin exposure resulted in selective loss of wild-type (mCherry_Cre) and strong expansion of knockout cells (GFP). Insertion site mapping of resistant cells (using Sanger Sequencing) confirmed disruption of porphyrin biosynthesis pathway components [Figure 1D]. These results ratify that mutations of enzymes of the porphyrin biosynthesis pathway induce artemisinin resistance in mouse ESCs.

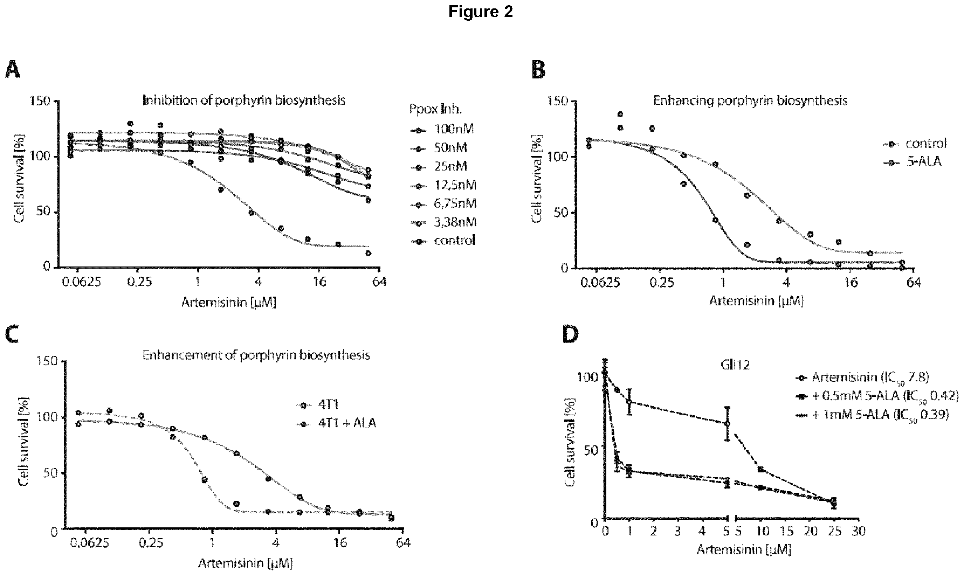

The applicant next assessed if modulation of cellular porphyrin biosynthesis is sufficient to alter susceptibility to artemisinin. The applicant used the pharmacological inhibitor of porphyrin production or heme biosynthesis, the protoporphyrinogen oxidase (PPOX) inhibitor acifluorfen, which blocks conversion of protoporphyrinogen IX to protoporphyrin IX, and observed increased resistance to artemisinin in mouse ESCs [Figure 2A] {Witkowski, D. A. & Halling, B. P. Inhibition of plant protoporphyrinogen oxidase by the herbicide acifluorfen-methyl. Plant Physiol. 90, 1239-1242 (1989)}. Conversely when the applicant enhanced porphyrin production using the non-proteinogenic amino acid and endogenous alanine analogue precursor δ-aminolevulinic acid (5-ALA), the applicant increased the sensitivity to the anti-malarial compound [Figure 2B]. The applicant next assayed if different murine and human cancer cell lines respond similarly to the combinatorial treatment, independent of basal growth and susceptibility rates [Figure 5B].

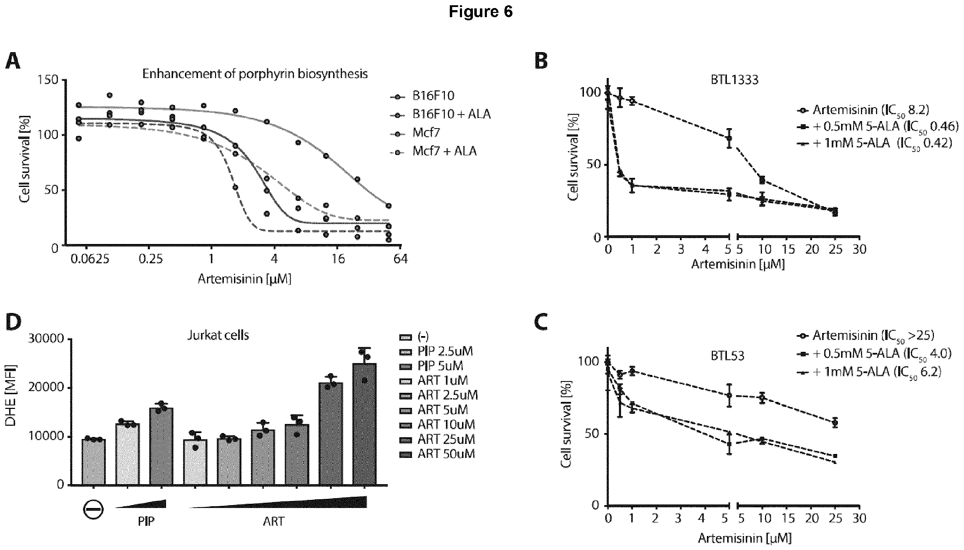

The applicant could not only confirm that all tested cancer cell gained hypersensitive to artemisinin [Figure 2C; Figure 6A]. However, supprisingly it was also observed 5-ALA induced artemisinin hypersensitivity to artemisinin cytotoxicity in three independently derived, clinically relevant human primary glioblastoma cell lines [Figure 2D; Figure 6B, 6C].

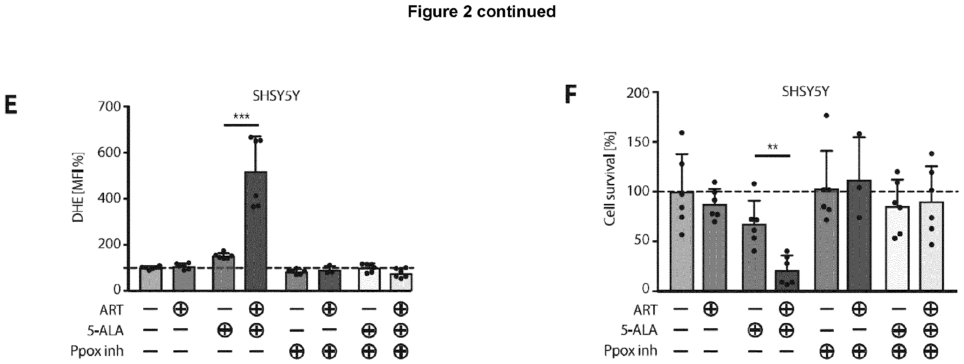

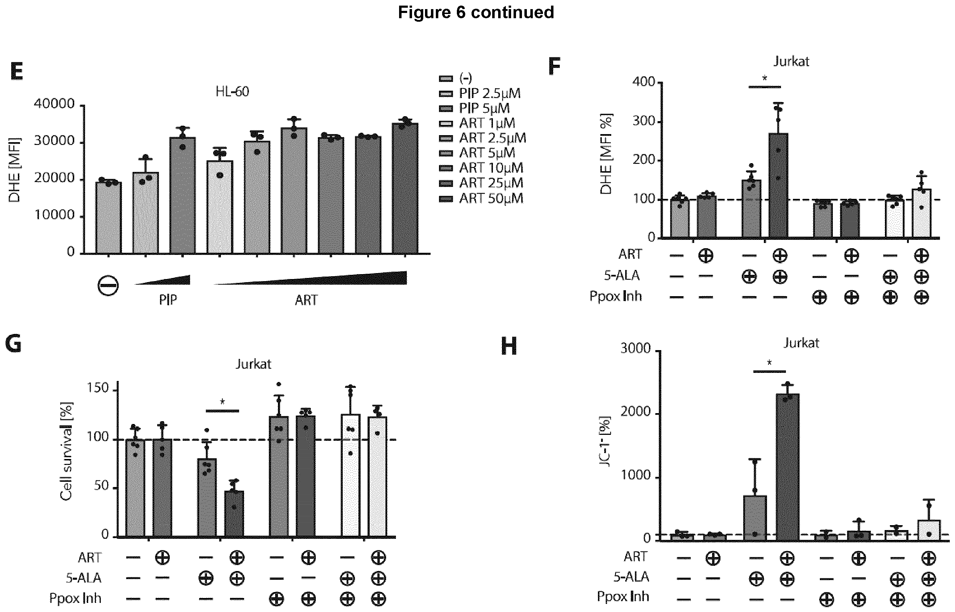

Mechanistically, artemisinin has been previously shown to cause reactive oxygen species (ROS) production {Gopalakrishnan, A. M. & Kumar, N. Antimalarial action of artesunate involves DNA damage mediated by reactive oxygen species. Antimicrob. Agents Chemother. 59, 317-325 (2015)} {Stockwin, L. H. et al. Artemisinin dimer anticancer activity correlates with heme-catalyzed reactive oxygen species generation and endoplasmic reticulum stress induction. Int. J. Cancer 125, 1266-1275 (2009)} {Berman, P. A. & Adams, P. A. Artemisinin enhances heme-catalysed oxidation of lipid membranes. Free Radic. Biol. Med. 22, 1283-1288 (1997).} Assessing ROS levels (using the redox-sensitive fluorescent probe Dihydroethidium, DHE) confirmed increased levels of ROS upon artemisinin treatment [Figure 6D, 6E]. The applicant next assessed if 5-ALA had any effet on ROS in presence of artemisinin. Notably, 5-ALA alone triggered ROS and slightly reduced cell viability. However, the combination of 5-ALA with artemisinin resulted in highly elevated ROS levels as well as a strong increase in cell death in two different cellular contexts [Figure 2E, 2F; Figure 6F, 6G]. Similarly, and previously associated with artemisinin induced ROS induction, mitochondrial polarisation (ΔΨm, as assessed by the mitochondrial membrane potential probe JC-1) was strongly elevated in combination with 5-ALA [Figure 6H] {Antoine, T. et al. Rapid kill of malaria parasites by artemisinin and semi-synthetic endoperoxides involves ROS-dependent depolarization of the membrane potential. J. Antimicrob. Chemother. 69, 1005-1016 (2014).}. Notably, 5-ALA treatment alone also increased mitochondrial polarisation indicating a sensitization for parallel artemisinin treatment. Importantly, all of the named phenotypes (ROS induction, mitochondrial depolarisation, cell death) could be supressed by pharmacological inhibition of porphyrin production [Figure 2E, 2F; Figure 6F, 6G, 6H]. These results demonstrated that artemisinin and 5-ALA induced elevated ROS levels (and mitochondrial membrane depolarisation) and cell death can be reverted by inhibition of porphyrin biosynthesis.

Altered cell metabolism and upregulation of porphyrin production is frequently observed in human cancer cells {Navone, N. M., Polo, C. F., Frisardi, A. L., Andrade, N. E. & Battle, A. M. Heme biosynthesis in human breast cancer--mimetic 'in vitro' studies and some heme enzymic activity levels. Int. J. Biochem. 22, 1407-1411 (1990).}. In human glioblastoma patients, elevated porphyrin biosynthesis is used therapeutically to localise and target cancer tissue, as porphyrin precursors (i.e. protoporphyrins) show strong fluorescence that can be monitored and functionally exploited {Zhao, S. et al. Intraoperative fluorescence-guided resection of high-grade malignant gliomas using 5-aminolevulinic acid-induced porphyrins: a systematic review and meta-analysis of prospective studies. PloS one 8, e63682 (2013)}. During tumor tissue resection in the clinics, oral or intravenous administration of 5-ALA is used to discriminate malignant tumor tissue from healthy brain matter, as fluorescent protoporphyrins, such as protoporphyrin IX, accumulate specifically in tumor cells {Marbacher, S. et al. Use of fluorescence to guide resection or biopsy of primary brain tumors and brain metastases. Neurosurg Focus 36, E10 (2014).}. The applicant thus assessed if endogenously altered porphyrin biosynthesis in brain cancer can be therapeutically exploited, using a combination of 5-ALA and artemisinin.

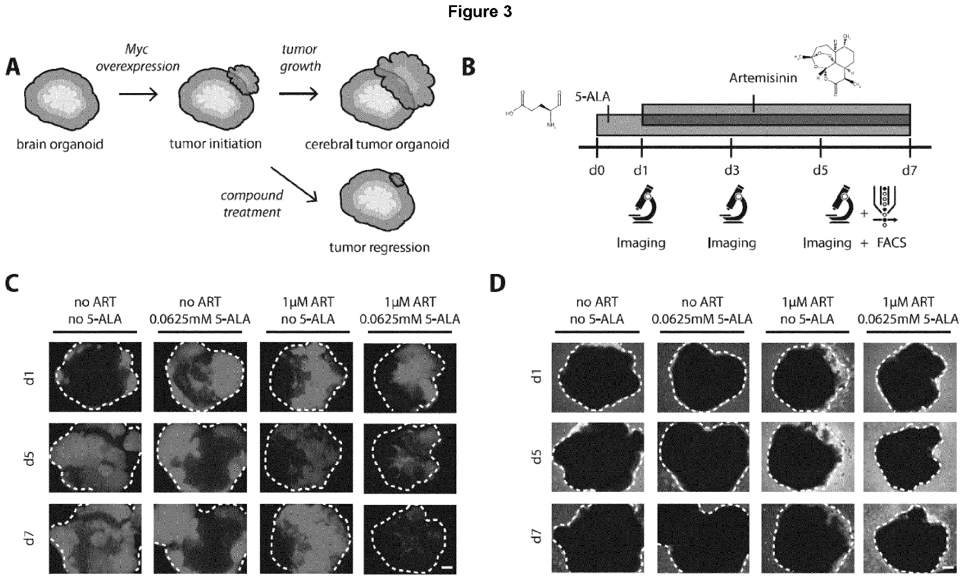

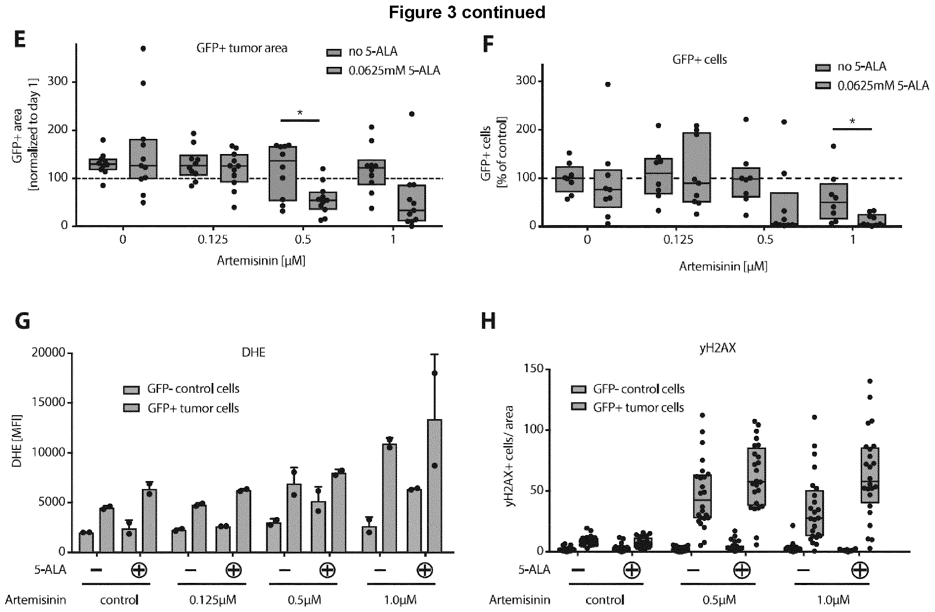

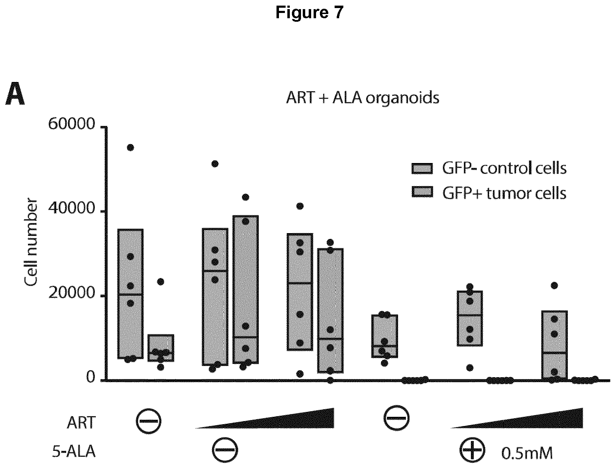

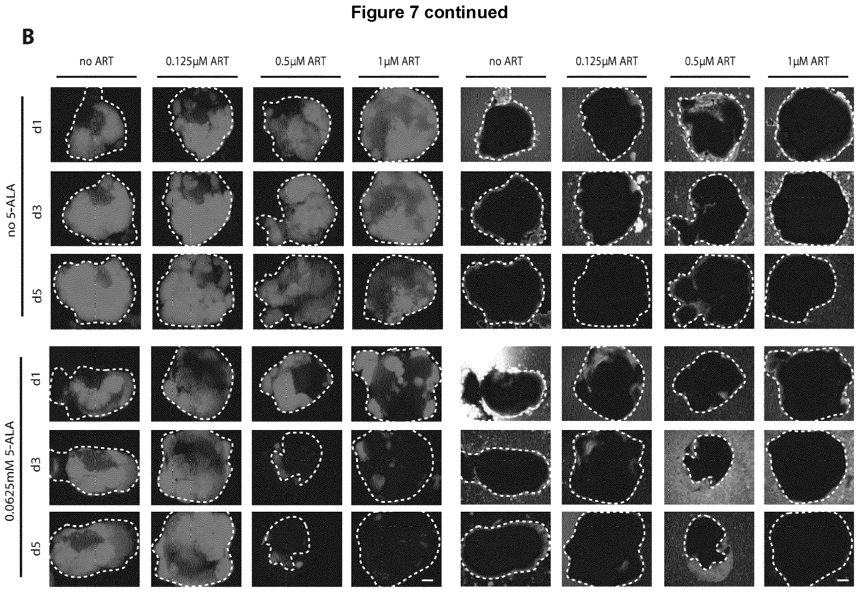

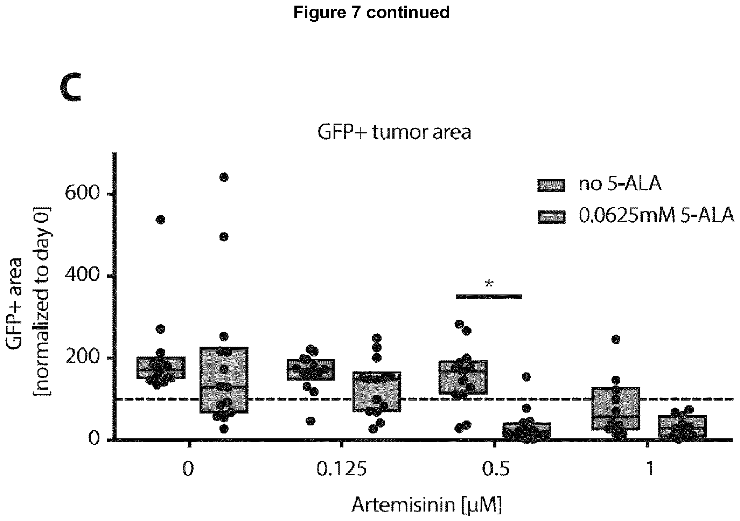

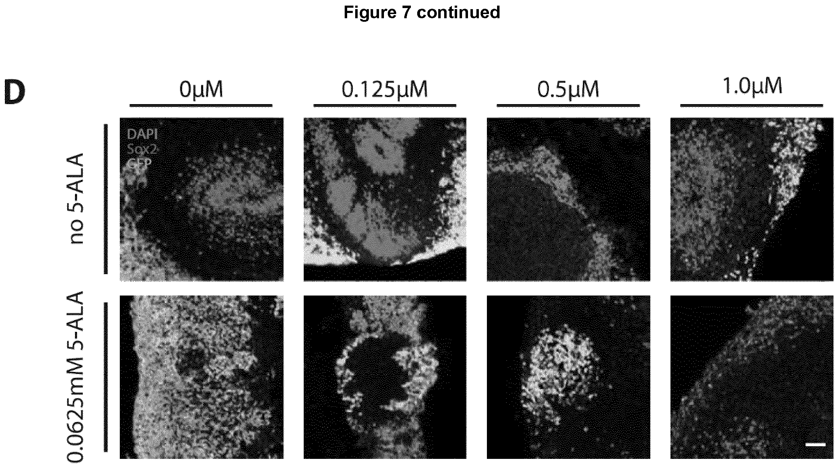

The applicant used a recently established human cerebral tumor organoid model, that is based on genetic manipulation of neuronal precursors in the course of brain organoid development, thus recapitulating important aspects of human tumor formation *in vitro* {Lancaster, M. A. et al. Cerebral organoids model human brain development and microcephaly. Nature 501, 373-379 (2013).}. Tumor cells are simultaneously labelled with GFP, allowing spatial monitoring over time and *in vitro* compound profiling in human cerebral tumor organoids [Figure 3A]. The applicant first assessed growth rates in presence of artemisinin and 5-ALA in a central nervous system primitive neuroectodermal tumor (CNS-PNET)-like neoplasm model, which is based on the overexpression of the c-MYC oncogene {Bian, S. et al. Genetically engineered cerebral organoids model brain tumor formation. Nat. Methods 15, 631-639 (2018)} [Figure 7A]. The highly malignant brain tumor organoids were treated with different dosages of 5-ALA, Artemisinin, or a combination of both [Figure 3B]. Since very high concentrations of 5-ALA or artemisinin alone resulted in notable growth inhibition of GFP-positive tumor organoids [Figure 7A], concentrations were chosen for all subsequent experiments where the individual compounds had no notable effects on tumor or organoid growth. The combination of both 5-ALA and artemisinin together markedly decreased GFP-positive tumor cells in human cerebral organoids [Figure 3C, 3D; Figure 7B]. Quantification of the tumor tissue area at different time points (d3, d5) [Figure 3E; Figure 7C] as well as FACS analysis (d5) [Figure 3F] confirmed reduction of the GFP-positive cells upon 5-ALA and artemisinin combination treatment. Immunohistochemistry of sectioned brain organoids further showed that 5-ALA treatment resulted in the losss of GFP+ tumor tissue, but had no apparent effect on the non-transformed neuronal tissues in the cerebral organoids, such as Sox2 expressing cells and/or rosette-like structures indicative or progenitor cells [Figure 7D, 7]. Altogether, this data suggests that 5-ALA and artemisinin combination therapy strongly reduces the number of tumor cells in a human *in vitro* primitive neuroectodemal tumor (PNET) organoid model.

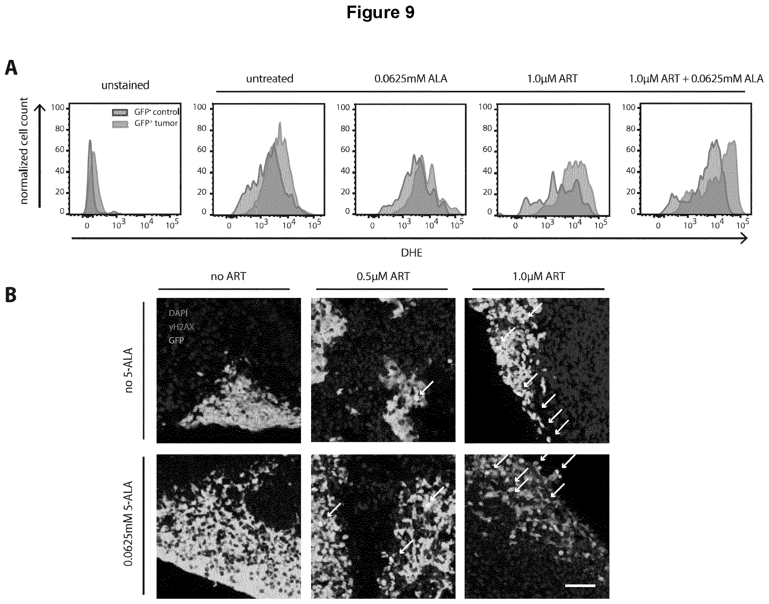

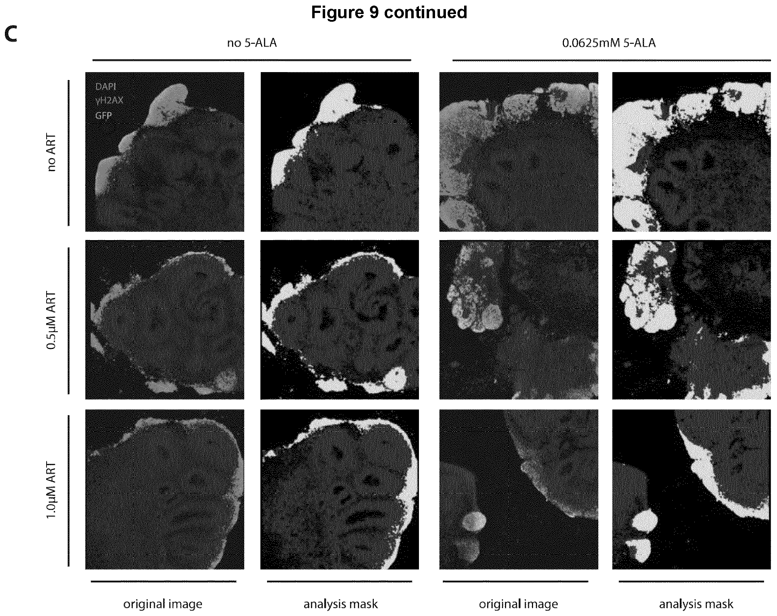

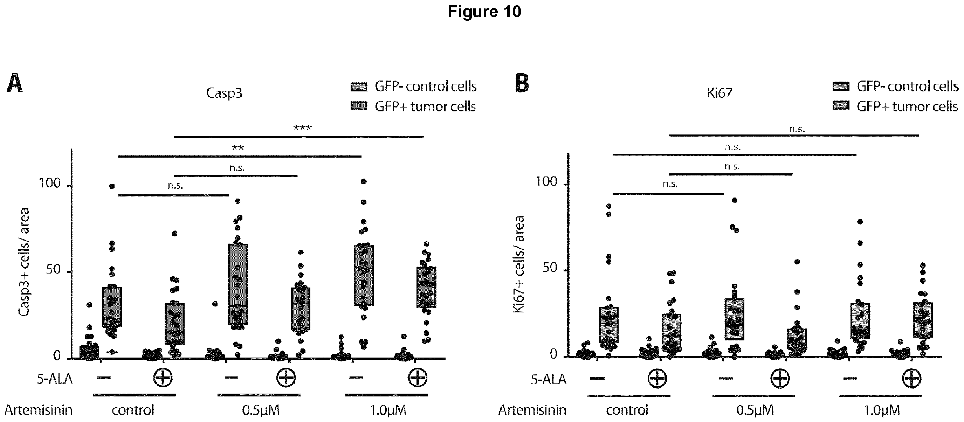

As the applicant observed a marked effect of 5-ALA and artemisinin on ROS levels, the applicant performed DHE staining and analysis of Myc overexpressing tumor organoids. Whereas GFP-positive tumor cells displayed slightly higher basal ROS signals as compared to GFP negative wild-type cells (as assessed by DHE staining and FACS analysis), ROS levels markedly increased further upon 5-ALA and artemisinin double treatment, especially in GFP-positive cancer cells [Figure 3G; Figure 9A]. Notably, 5-ALA treatment alone also slightly induced ROS production in this setup, sensitizing for artemisinin toxicity. As elevated intracellular ROS also causes wide-spread protein and DNA damage, the applicant assessed the amount of DNA double strand breaks (DSB) on sections of treated organoids, and could indeed observe a very strong increase in γH2AX (phosphorylated histone H2AX)-positive cells in 5-ALA and artemisinin double-treated GFP-positive tumor tissue, but not in wild-type cells [Figure 3H; Figure 9B, 9C]. Additionally, while the basal number of apoptotic (Caspase 3-positive) and proliferating cells (Ki67-positive) was increased in GFP-positive tumor cells as compared to normal tissue, apoptosis further increased in GFP/positive tumor cells upon combination treatment with artemisinin and 5-ALA [Figure 10A-C].

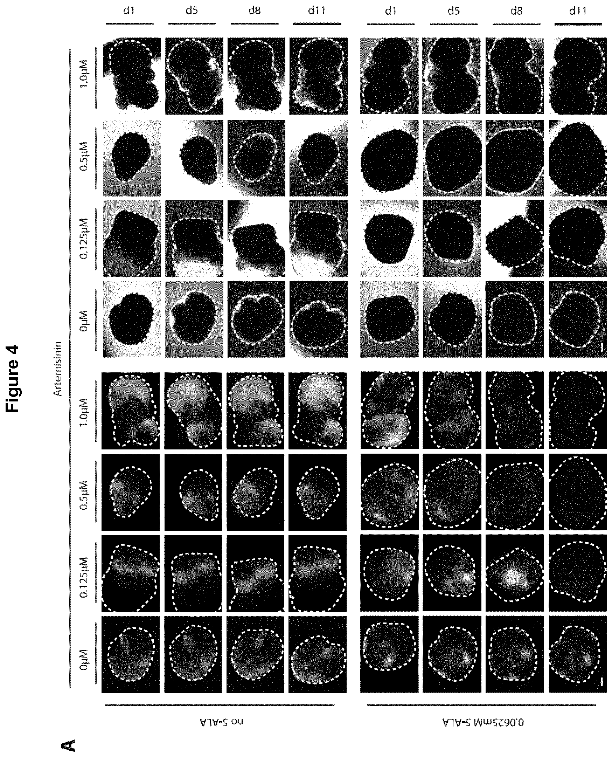

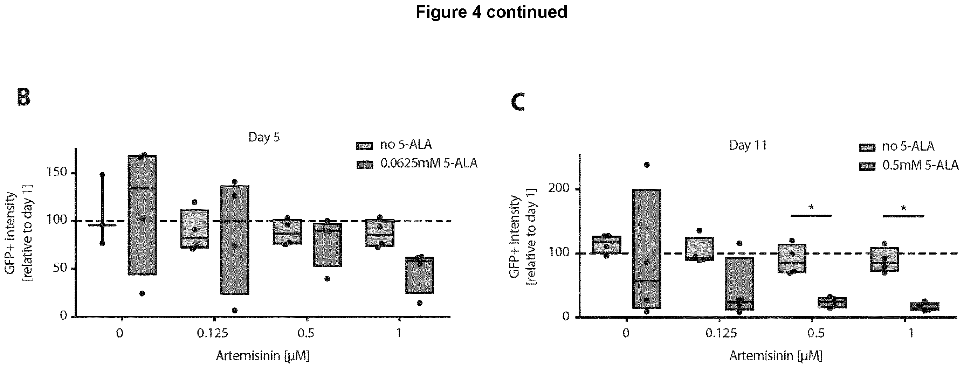

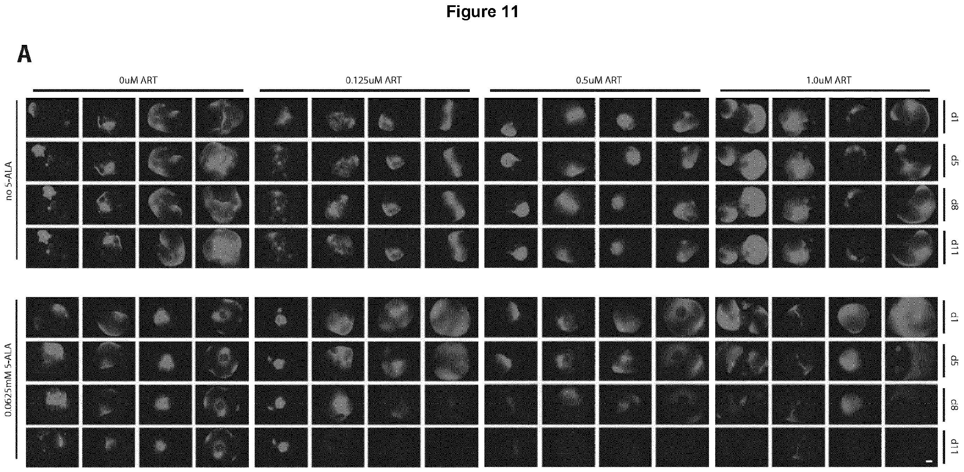

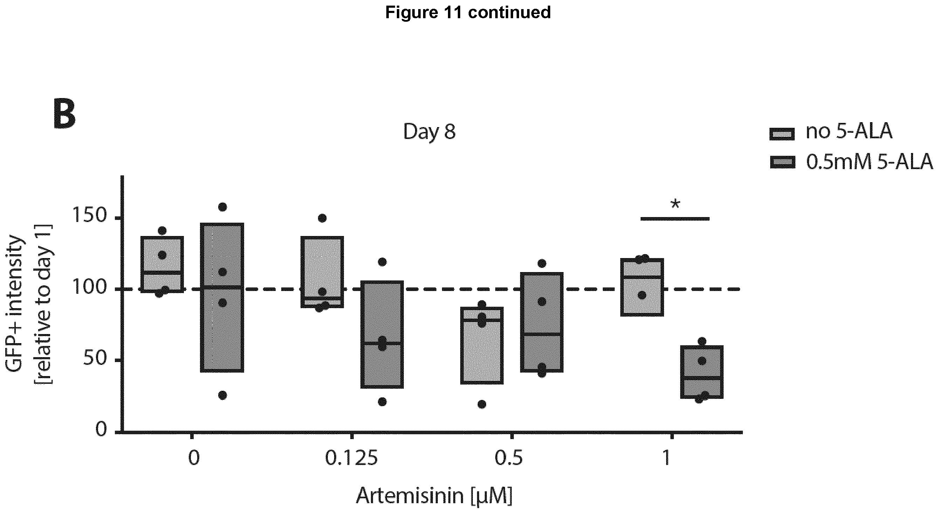

As 5-ALA is used clinically for the diagnosis and therapy of human glioblastomas, the applicant assessed 5-ALA and artemisinin combination treatment in an *in vitro* human glioblastoma-like neoplastic organoid model {Bian, S. et al. Genetically engineered cerebral organoids model brain tumor formation. Nat. Methods 15, 631-639 (2018)}. These organoids were engineered to carry mutations of the tumor suppressor genes p53, NF1 and PTEN, together with GFP with allowed us to monitor tumor growth over time in presence of Artemisinin, 5-ALA or a combination of both. Whereas both 5-ALA and Artemisinin alone had little effect on the survival of untransformed cells as well as transformed cells, the combination of both compounds specifically ablated GFP-positive tumor cells in the *in vitro* tumor model [Figure 4A; Figure 11A]. Quantification and image analysis of cerebral organoids confirmed progressive loss of GFP-positive tumor cells and tissue, but not untransformed ones [Figure 4B;4C Figure 11B, 11C]. These data show that 5-ALA and artemisinin lead to increased cell death of tumor cells in two highly malignant brain tumor models, via elevated ROS levels and increased DNA damage.

In summary, the applicant unambiguously delineates the requirement of porphyrin biosynthesis as the critical endogenous pathway for artemisinin-triggered cytotoxicity in eukaryotic cells. Using a high-throughput genetic screening system, the applicant has identified mitochondrial function and more specifically porphyrin/heme biosynthesis, to be required for the activity of this anti-malarial and anti-cancer compound. Genetic as well as pharmacological modulation of porphyrin production was sufficient to modulate artemisinin toxicity, as well as control the levels of artemisinin-induced reactive oxygen species, in multiple cellular contexts and different species. Notably, heme biosynthesis induction alone slightly elevated cellular ROS levels, sensitizing for artemisinin-induced toxicity. Furthermore, the applicant shows in several independent *in vitro* human cerebral tumor organoid and spheroid model systems, that the applicant can specifically target brain cancer cells, in particular, human glioblastomas, using combination treatment of 5-ALA and artemisinin to induce cell death.

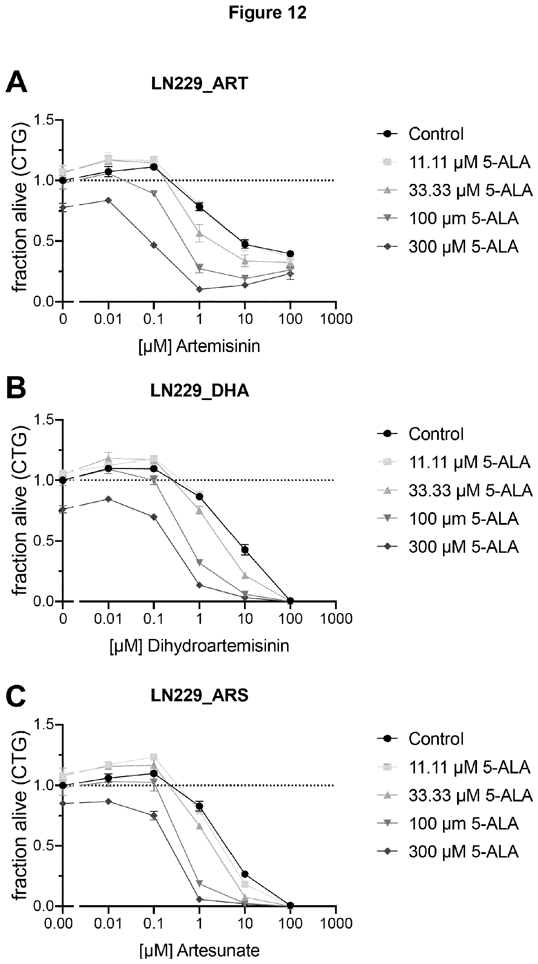

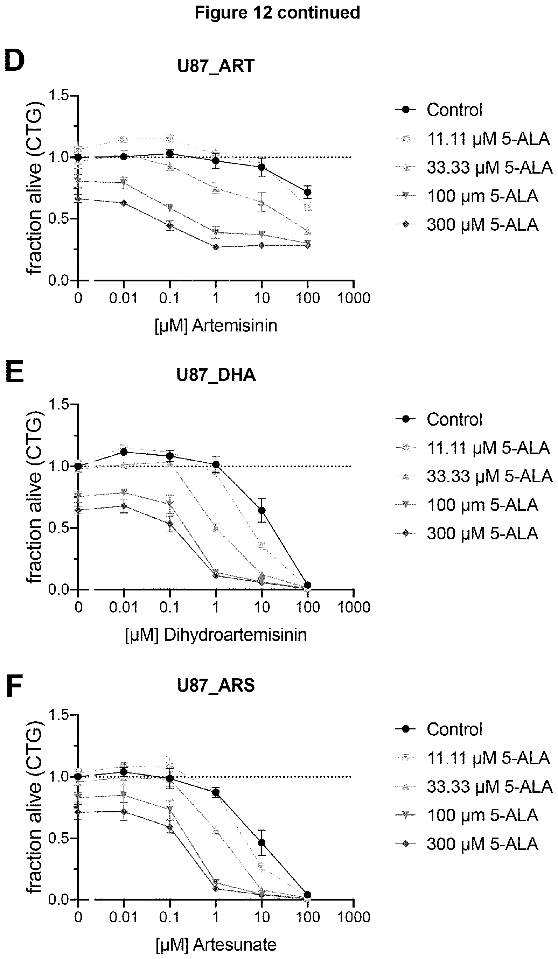

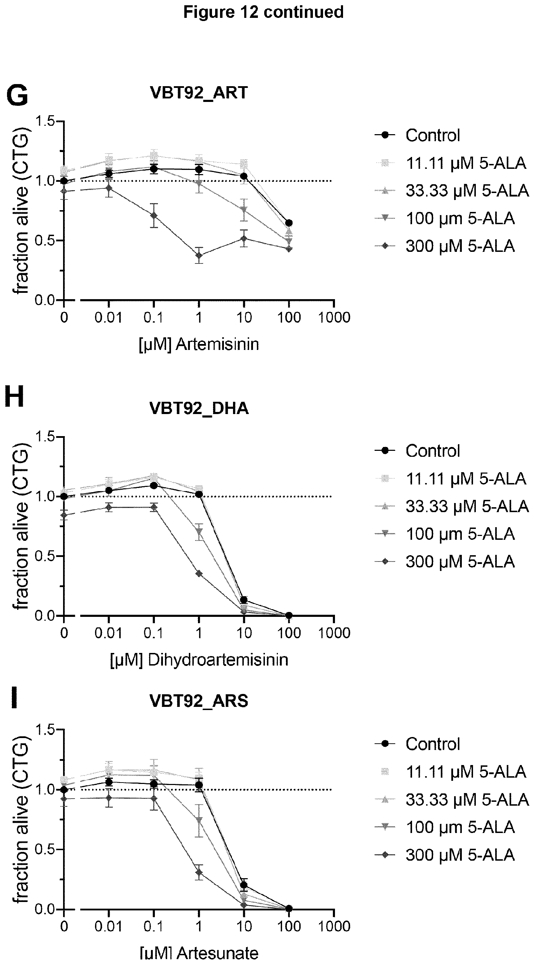

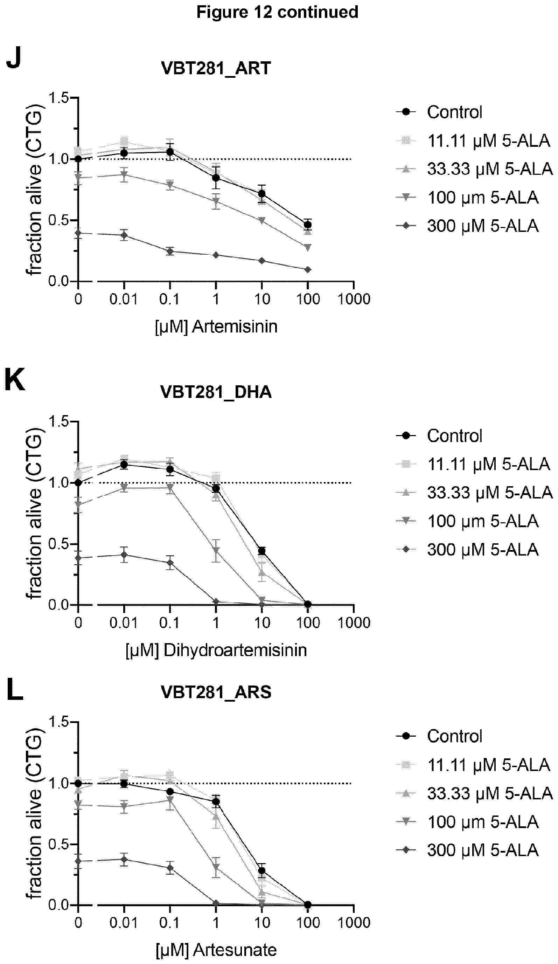

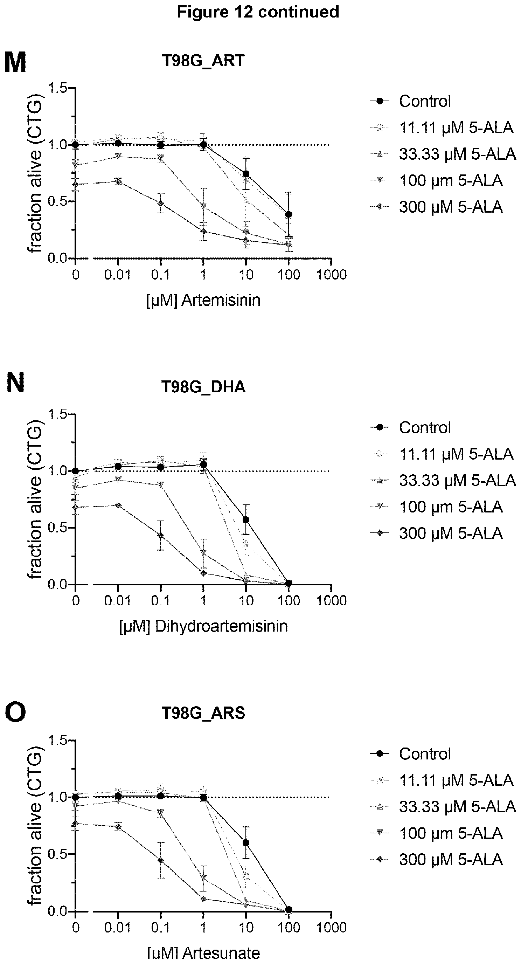

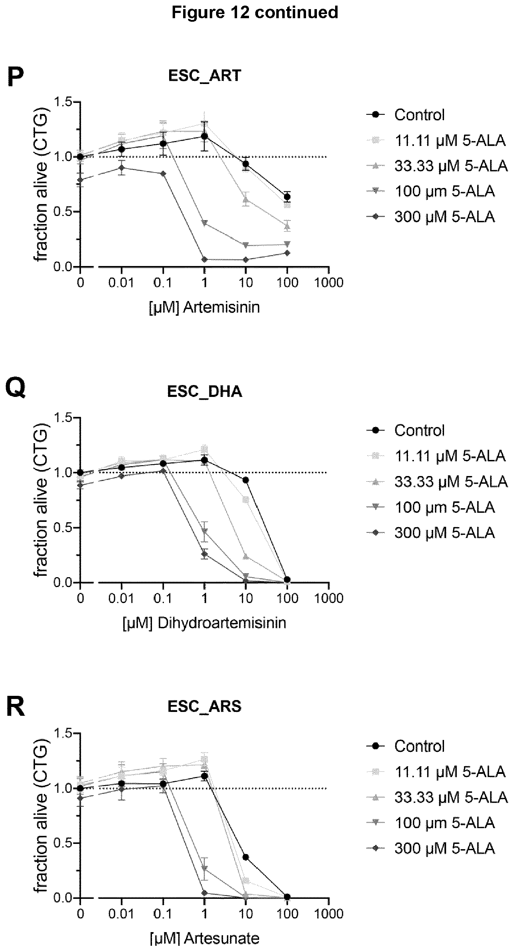

After demonstrating a strong synergy of artemisinin (ART) and 5-aminolevulinic acid (5-ALA) for the treatment of brain cancer, the applicant next assessed whether this synergy extends to other ART derivates, additional cancer types, and whether this double combination is synergistic to currently applied cancer treatment regiments, particularly chemotherapy. Using a 6-day viability assay the applicant confirmed that ART itself and the most commonly used ART derivates, dihydroartemisinin (DHA) and artesunate (ARS), in combination with 5-ALA show strongly synergistic, antiproliferative effects on multiple glioblastoma cell lines as well as the highly proliferative mouse embryonic stem cells (ESC, Figure 12). From these data the applicant derived cell-type and compound-specific dose combinations for the following analyses.

Glioblastoma multiforme is a highly aggressive cancer currently treated by maximal surgery resection, followed by radiotherapy plus concomitant and maintenance temozolomide (TMZ) treatment. The inventors have shown that combination of TMZ with 5-ALA plus ART or DHA is more effective than all possible double combinations in all glioblastoma lines tested (Figure 13). In ESC for example, only the double combination of DHA and 5-ALA elicits a cytotoxic effect of TMZ, indicating clear synergy.

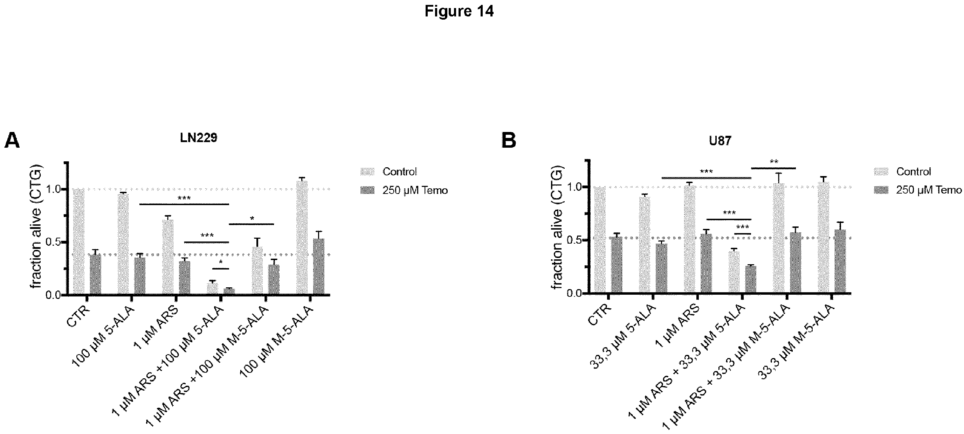

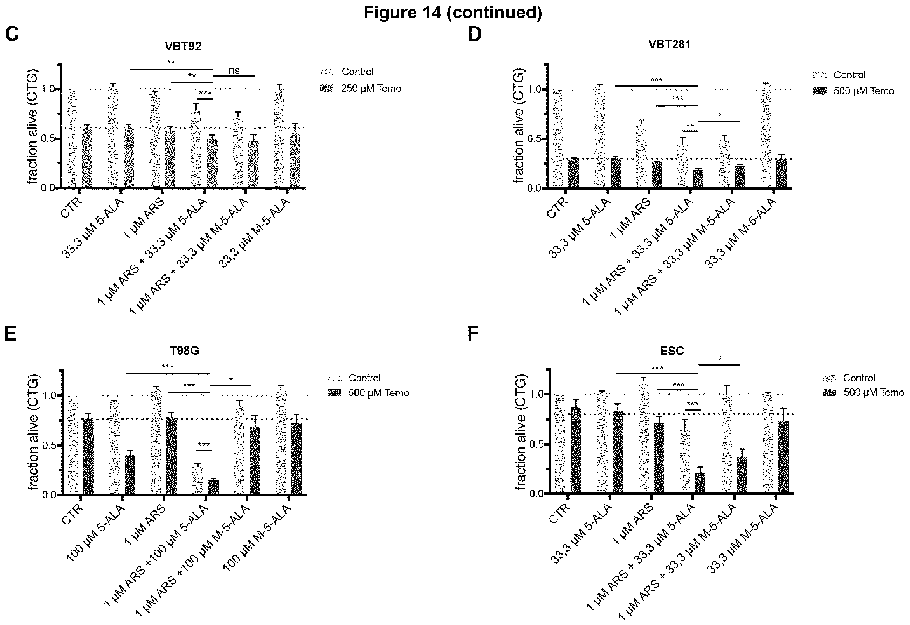

These findings extend to ARS and the combination of ARS with 5-ALA and TMZ is more effective than replacing 5-ALA by its derivative Methyl-5-ALA (M-5-Ala) in 4 out of 5 glioblastoma lines tested (Figure 14).

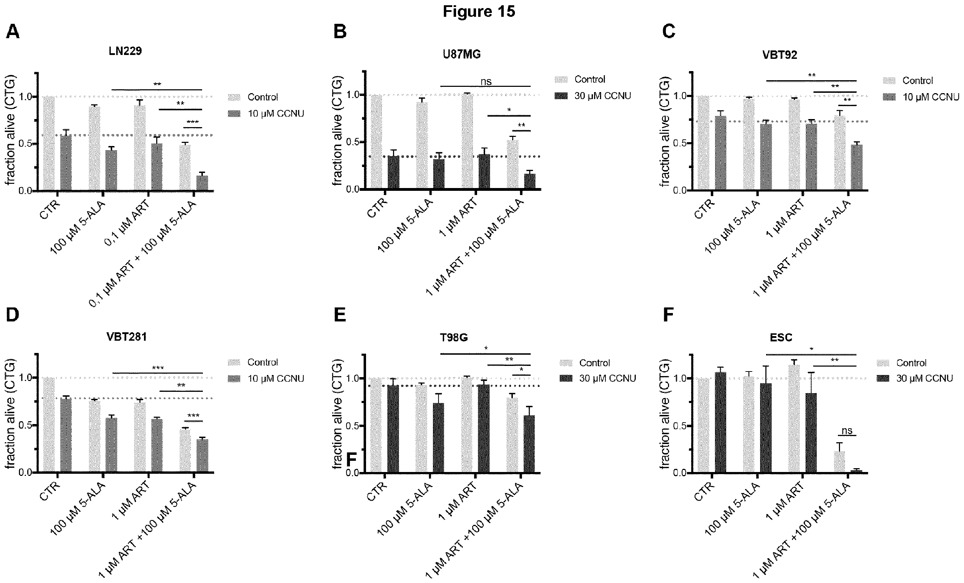

Lomustine (CCNU) is an alternative to TMZ in the treatment of brain cancers and, like for TMZ, its cytostatic effects can be particularly strengthened by combination with ART plus 5-ALA as compared to all possible double combinations (Figure 15).

Moreover, the inventors tested the combination of ART and derivates with 5-ALA in cancer cell lines originating from other tissues. Not only the inventors found a strong synergy of ART/DHA/ARS with 5-ALA in reducing the survival of these cancer lines, but also this double combination enhances the widely used chemotherapeutic agents cisplatin (CP), carboplatin, triptolide, homoharringtonin, dactinomycin, doxorubicin, epirubicin, vinorebine, temozolomide, lomustine, and 5-fluorouracil (5-FU). This was particularly tested for two lung cancer cell lines (Figure 16), HepG2 cells (liver, Figure 17), MD-MBA-231 cells (breast, Figure 18), and MiaPaca-2 cells (pancreas, Figure 19). Collectively, the inventor's data for the first time show the strong potential of ART and ART-derivatives treatment in combination with 5-ALA and currently used chemotherapeutic agents and especially an anti-glioblastoma drug in, for instance, hematopoietic cancers, brain cancer, lung cancer, liver cancer, breast cancer, and pancreatic cancer.

Thus, the present invention relates to a pharmaceutical composition comprising
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof; and
b) 5-aminolevulinic acid (**2**), methyl-5-aminolevulinic acid (**2b**), or a pharmaceutically acceptable salt or solvate thereof; and
c) at least one chemotherapeutic agent, as mentioned in the claims, for use in prophylaxis and/or treatment of hematopoietic cancers, brain cancer, pancreatic cancer, liver cancer, breast cancer, and lung cancer and especially glioblastoma.

Preferably, the present invention relates to a pharmaceutical composition comprising
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof; and
b) 5-aminolevulinic acid (**2**) or a pharmaceutically acceptable salt or solvate thereof; and
c) at least one chemotherapeutic agent, as mentioned in the claims, for use in prophylaxis and/or treatment of hematopoietic cancers, brain cancer, pancreatic cancer, liver cancer, breast cancer, and lung cancer and especially glioblastoma.

Still more preferably, the present invention relates to a pharmaceutical composition comprising
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof; and
b) 5-aminolevulinic acid (**2**) or a pharmaceutically acceptable salt or solvate thereof; and
c) at least one anti-glioblastoma drug selected from the group consisting of temozolomide, lomustine, cisplatin, and 5-fluorouracil and most preferred is temozolomide for use in prophylaxis and/or treatment of hematopoietic cancers, brain cancer, pancreatic cancer, liver cancer, breast cancer, and lung cancer and especially glioblastoma.

For use in prophylaxis and/or treatment of a cancer selected from hematopoietic cancers, brain cancer, pancreatic cancer, liver cancer, breast cancer, and lung cancer such as non-small cell lung cancer, preferably, brain cancer, more preferably, glioblastoma, a molar ratio of the artemisinin compound and the 5-aminolevulinic acid is in a range of 1:5 to 1:5000, preferably 1:5 to 1:1000, more preferably 1:5 to 1:500, still more preferably 1:10 to 1:250, most preferably 1:10 to 1:100 in the pharmaceutical composition.

For use in prophylaxis and/or treatment of a cancer selected from hematopoietic cancers, brain cancer, pancreatic cancer, liver cancer, breast cancer, and lung cancer such as non-small cell lung cancer, preferably, brain cancer, more preferably, glioblastoma, a molar ratio of the artemisinin compound and the methyl-5-aminolevulinic acid is in a range of 1:5 to 1:5000, preferably 1:5 to 1:1000, more preferably 1:5 to 1:500, still more preferably 1:10 to 1:250, most preferably 1:10 to 1:100 in the pharmaceutical composition.

The pharmaceutical composition according to the invention comprise an artemisinin compound or a pharmaceutically acceptable salt, cocrystal, solvate thereof and 5-aminolevulinic acid or methyl-5-aminolevulinic acid and preferably 5-aminolevulinic acid and the chemotherapeutic agent, preferably the anti-glioblastoma drug as only active ingredients especially for the treatment of hematopoietic cancers, brain cancer, pancreatic cancer, liver cancer, breast cancer, and lung cancer such as non-small cell lung cancer, preferably brain cancer and more preferably glioblastoma.

In another aspect, the present invention relates to a method for the prophylaxis and/or treatment of a cancer selected from hematopoietic cancers, brain cancer, pancreatic cancer, liver cancer, breast cancer, and lung cancer, and especially glioblastoma comprising administering to a patient in need thereof a therapeutically effective amount of the pharmaceutical composition comprising
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof; and
b) 5-aminolevulinic acid (**2**), methyl-5-aminolevulinic acid (**2b**), or a pharmaceutically acceptable salt or solvate thereof; and
c) at least one chemotherapeutic agent, as defined in the claims.

Furthermore, the present invention relates to a method for the prophylaxis and/or treatment of a cancer selected from hematopoietic cancers, brain cancer, pancreatic cancer, liver cancer, breast cancer, and lung cancer, and especially glioblastoma comprising administering to a patient in need thereof a therapeutically effective amount of the pharmaceutical composition comprising
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof; and
b) 5-aminolevulinic acid (**2**) or a pharmaceutically acceptable salt or solvate thereof; and
c) at least one chemotherapeutic agent, as defined in the claims.

Still more preferably, the present invention relates to a method for the prophylaxis and/or treatment of a cancer selected from hematopoietic cancers, brain cancer, pancreatic cancer, liver cancer, breast cancer, and lung cancer, and especially glioblastoma comprising administering to a patient in need thereof a therapeutically effective amount of the pharmaceutical composition comprising
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof; and
b) 5-aminolevulinic acid (**2**) or a pharmaceutically acceptable salt or solvate thereof; and
c) at least one anti-glioblastoma drug selected from the group consisting of temozolomide, lomustine, cisplatin, and 5-fluorouracil and most preferred is temozolomide.

Optionally, for use in prophylaxis and/or treatment of a cancer selected from hematopoietic cancers, brain cancer, pancreatic cancer, liver cancer, breast cancer, and lung cancer such as non-small cell lung cancer, preferably, brain cancer, more preferably, glioblastoma, the pharmaceutical composition according to the present invention further comprises pharmaceutically acceptable carrier, excipient and/or diluent. The pharmaceutical compositions of the present invention can be prepared in a conventional solid or liquid carrier or diluent and a conventional pharmaceutically made adjuvant at suitable dosage level in a known way.

The pharmaceutical compositions according to the present invention will typically be administered together with suitable acceptable carrier selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the derivatives according to the general formula (I) or analogues compound thereof or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. anti-cancer activity or activity against cancer metastases and the like. Suitable dosage forms for sustained release include tablets having layers of varying disintegration rates or controlled release, polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to 1 weight %.

Thus, the present invention provides a pharmaceutical composition comprising or consisting of
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one chemotherapeutic agent, as mentioned in the claims, and
d) a pharmaceutically acceptable carrier, excipient and/or diluent.

More preferably the present application relates to a pharmaceutical composition comprising or consisting of
a) artemisinin (**1a**) or artesunate (**1e**),
   or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one chemotherapeutic agent, as mentioned in the claims, and
d) a pharmaceutically acceptable carrier, excipient and/or diluent.

Still more preferably the present application relates to a pharmaceutical composition comprising or consisting of
a) artesunate (**1e**) or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one chemotherapeutic agent, as mentioned in the claims, and
d) a pharmaceutically acceptable carrier, excipient and/or diluent.

Still more preferably the present application relates to a pharmaceutical composition comprising or consisting of
a) artemisinin (**1a**) or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one chemotherapeutic agent, as mentioned in the claims, and
d) a pharmaceutically acceptable carrier, excipient and/or diluent.

Moreover, the present application relates preferably to a pharmaceutical composition comprising or consisting of
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof; and
b) 5-aminolevulinic acid (**2**) or a pharmaceutically acceptable salt or solvate thereof; and
c) at least one chemotherapeutic agent, as mentioned in the claims, and
d) a pharmaceutically acceptable carrier, excipient and/or diluent.

More preferably the present application relates to a pharmaceutical composition comprising or consisting of
a) artemisinin (**1a**) or artesunate (**1e**),
   or a pharmaceutically acceptable salt, cocrystal, or solvate thereof; and
b) 5-aminolevulinic acid (**2**) or a pharmaceutically acceptable salt or solvate thereof; and
c) at least one chemotherapeutic agent, as mentioned in the claims, and
d) a pharmaceutically acceptable carrier, excipient and/or diluent.

Still more preferably the present application relates to a pharmaceutical composition comprising or consisting of
a) artesunate (**1e**) or a pharmaceutically acceptable salt, cocrystal, or solvate thereof; and
b) 5-aminolevulinic acid (**2**) or a pharmaceutically acceptable salt or solvate thereof; and
c) at least one chemotherapeutic agent, as mentioned in the claims, and
d) a pharmaceutically acceptable carrier, excipient and/or diluent.

Still more preferably the present application relates to a pharmaceutical composition comprising or consisting of
a) artemisinin (**1a**) or a pharmaceutically acceptable salt, cocrystal, or solvate thereof; and
b) 5-aminolevulinic acid (**2**) or a pharmaceutically acceptable salt or solvate thereof; and
c) at least one chemotherapeutic agent, as mentioned in the claims, and
d) a pharmaceutically acceptable carrier, excipient and/or diluent.

Furthermore, the present application relates to a pharmaceutical composition comprising or consisting of
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one anti-glioblastoma drug selected from the group consisting of temozolomide, lomustine, cisplatin, and 5-fluorouracil; most preferred is temozolomide; and
d) a pharmaceutically acceptable carrier, excipient and/or diluent.

More preferably the present application relates to a pharmaceutical composition comprising or consisting of
a) artemisinin (**1a**) or artesunate (**1e**),
   or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one anti-glioblastoma drug selected from the group consisting of temozolomide, lomustine, cisplatin, and 5-fluorouracil; most preferred is temozolomide; and
d) a pharmaceutically acceptable carrier, excipient and/or diluent.

Still more preferably the present application relates to a pharmaceutical composition comprising or consisting of
a) artesunate (**1e**) or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one anti-glioblastoma drug selected from the group consisting of temozolomide, lomustine, cisplatin, and 5-fluorouracil; most preferred is temozolomide; and
d) a pharmaceutically acceptable carrier, excipient and/or diluent.

Still more preferably the present application relates to a pharmaceutical composition comprising or consisting of
a) artemisinin (**1a**) or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one anti-glioblastoma drug selected from the group consisting of temozolomide, lomustine, cisplatin, and 5-fluorouracil; most preferred is temozolomide; and
d) a pharmaceutically acceptable carrier, excipient and/or diluent.

Furthermore, the present application relates preferably to a pharmaceutical composition comprising or consisting of
a) an artemisinin compound (**1**) selected from the group consisting of artemisinin (**1a**), dihydroarteminisin (**1b**), and artesunate (**1e**),
   or a pharmaceutically acceptable salt, cocrystal, or solvate thereof;
b) 5-aminolevulinic acid (**2**) or a pharmaceutically acceptable salt or solvate thereof; and
c) at least one anti-glioblastoma drug selected from the group consisting of temozolomide, lomustine, cisplatin, and 5-fluorouracil; most preferred is temozolomide; and
d) a pharmaceutically acceptable carrier, excipient and/or diluent.

More preferably the present application relates to a pharmaceutical composition comprising or consisting of
a) artemisinin (**1a**) or artesunate (**1e**),
   or a pharmaceutically acceptable salt, cocrystal, or solvate thereof;
b) 5-aminolevulinic acid (**2**) or a pharmaceutically acceptable salt or solvate thereof; and
c) at least one anti-glioblastoma drug selected from the group consisting of temozolomide, lomustine, cisplatin, and 5-fluorouracil; most preferred is temozolomide; and
d) a pharmaceutically acceptable carrier, excipient and/or diluent.

Still more preferably the present application relates to a pharmaceutical composition comprising or consisting of
a) artesunate (**1e**) or a pharmaceutically acceptable salt, cocrystal, or solvate thereof;
b) 5-aminolevulinic acid (**2**) or a pharmaceutically acceptable salt or solvate thereof; and
c) at least one anti-glioblastoma drug selected from the group consisting of temozolomide, lomustine, cisplatin, and 5-fluorouracil; most preferred is temozolomide; and
d) a pharmaceutically acceptable carrier, excipient and/or diluent.

Still more preferably the present application relates to a pharmaceutical composition comprising or consisting of
a) artemisinin (**1a**) or a pharmaceutically acceptable salt, cocrystal, or solvate thereof;
b) 5-aminolevulinic acid (**2**) or a pharmaceutically acceptable salt or solvate thereof; and
c) at least one anti-glioblastoma drug selected from the group consisting of temozolomide, lomustine, cisplatin, and 5-fluorouracil; most preferred is temozolomide; and
d) a pharmaceutically acceptable carrier, excipient and/or diluent.

The pharmaceutical composition according to the present invention may be admistered in combination with a radiotherapy, immunotherapy, electromagnetic field therapy, hyperthermia therapy, chemotherapy, cancer immunotherapy and/or a small molecule based therapy.

In some embodiments, the pharmaceutical composition according to the present invention is used for prophylaxis and/or treatment of hematopoietic cancers, brain cancer, pancreatic cancer, liver cancer, breast cancer, and lung cancer such as non-small cell lung cancer, preferably brain cancer, more preferably, glioblastoma preferably in combination with only one chemotherapeutic agent, preferably only one anti-glioblastoma drug, wherein the one chemotherapeutic agent, preferably the one anti-glioblastoma drug is selected from temozolomide.

As used herein "combination" or "pharmaceutical combination" means a product that results from the mixing or combining of more than one active ingredient and includes both fixed and non-fixed combinations of the active ingredients.

The term "fixed combination" or "fixed dose" means that the active ingredients, e.g. a compound of formula 1 and a combination partner, i.e. an anticancer agent, are both administered to a patient simultaneously in the form of a single entity or dosage. In other terms: the active ingredients are present in one dosage form, e.g. in one tablet or in one capsule.

The term "non-fixed combination" means that the active ingredients, e.g. a compound of formula 1 and a combination partner, i.e. an anticancer agent, are both administered to a patient as separate entities either simultaneously, concurrently or sequentially with no specific time limits, wherein such administration provides therapeutically effective levels of the two compounds in the body of a mammal or human in need thereof. The latter also applies to cocktail therapy, e.g. the administration of three or more anticancer agents.

Said pharmaceutical combination may be administered independently at the same time or separately within time intervals, especially here these time intervals allow that the combination partners show a synergistic effect.

The term "synergistic effect" means a therapeutic effect observed following administration of two or more active ingredients (e.g., one artemisinin compound, 5-ALA, at least one chemotherapeutic agent or preferably the at least one anti-glioblastoma drug) that is greater than the effect obtained by the administration of each single active ingredient. Thus, a synergistic effect is present when the effect obtained is larger than just the additional effect expected by the co-administration of the active ingredients.

By "syngergistic increase" is meant the combination of two or more active ingredients (e.g., one artemisinin compound, 5-ALA, at least one anti-glioblastoma drug) that results in an increase in cancer cell death that exceeds just an additional effect expected by the co-administration of the active ingredients.

By "synergistic decrease" is meant the combination of two or more active ingredients (e.g., one artemisinin compound, 5-ALA, at least one anti-glioblastoma drug) that results in a decrease in one or more symptoms of a cancer that is greater than just an additional effect expected by the co-administration of the active ingredients.

In another example of synergy, a therapeutic effect is observed for the combination of two or more active ingredients, wherein one or more of the active ingredients are present at a dose that is normally non-therapeutic. In another example of synergy, the combination of two or more active ingredients results in an unexpected decrease in toxicity (i.e., a level of toxicity that is less than the sum of the toxicity observed following administration of the single agents).

However, a synergistic effect can also be obtained by combining the administration of the pharmaceutical composition according to the present invention with a treatment by radiotherapy, immunotherapy, electromagnetic field therapy, hyperthermia therapy, chemotherapy, cancer immuntherapy or, as outlined above, any other small molecule based therapy.

In some embodiments, the pharmaceutical composition is used in a form of tablet, capsule, syrup, solution, suspension, emulsion, or gel.

In some embodiments, for prophylaxis and/or treatment of hematopoietic cancers, brain cancer, pancreatic cancer, liver cancer, breast cancer, and lung cancer such as non-small cell lung cancer, preferably brain cancer, more preferably, glioblastoma, the pharmaceutical composition is administered by oral or parenteral application.

The preferred preparations are adapted for oral application. These administration forms include, for example, pills, tablets, film tablets, coated tablets, capsules, powders and deposits.

The parenteral application includes dermal, intradermal, intragastral, intracutaneous, intravasal, intravenous, intramuscular, intraperitoneal, intranasal, intravaginal, intrabuccal, percutan, rectal, subcutaneous, sublingual, topical, or transdermal application. Preferably, the parenteral application includes intradermal, intragastral, intracutaneous, intravasal, intravenous, intramuscular, subcutaneous, sublingual, topical, transdermal application and inhalation.

The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatines or starch for holding or containing compositions comprising the active ingredient(s). Capsules with hard shells are typically made of blended of relatively high gel strength gelatines from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semisolid matrix. Powders for constitution refers to powder blends containing the active ingredients and suitable diluents which can be suspended e.g. in water or in juice.

Moreover, the pharmaceutical compositions of the present invention may be formulated in sustained release form to provide the rate controlled release of any one or more of the components or active ingredients to optimise the therapeutic effect(s), e.g. anticancer activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

In some embodiments, for prophylaxis and/or treatment of glioblastoma, the artemisinin compound, in particular artemisinin (**1a**), contained in the pharmaceutical composition is administered in a range of 0.01 to 100 mg/kg, preferred, 0.1 to 100 mg/kg, more preferred 0.1 to 50 mg/kg, most preferred 0.1 to 15 mg/kg per body weight per day and the 5-aminolevulinic acid is administered in a range of 0.01 to 200 mg/kg, preferred, 0.1 to 200 mg/kg, more preferred 0.1 to 50 mg/kg, most preferred 1 to 50 mg/kg per body weight per day.

Preferred, the dose of the invention regarding the artemisinin compound, in particular artemisinin is administered at least 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1.00, 1.05, 1.10, 1.15, 1.20, 1.25, 1.30, 1.35, 1.40, 1.45, 1.50, 2.00, 2.50, 3.00, 3.50, 4.00, 4.50, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5 or 15.0 mg/kg artemisinin per body weight per day.

Preferred, the dose of the invention regarding 5-aminolevulinic acid is administered at least 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1.00, 1.05, 1.10, 1.15, 1.20, 1.25, 1.30, 1.35, 1.40, 1.45, 1.50, 2.00, 2.50, 3.00, 3.50, 4.00, 4.50, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 17.0, 20.0, 25.0, 30.0, 35.0, 40.0, 45.0, or 50.0 mg/kg 5-aminolevulinic acid per body weight per day.

In some embodiments, the pharmaceutical composition is used for prophylaxis and/or treatment of glioblastoma preferably in combination with the at least one chemotherapeutic agent, preferably at least one anti-glioblastoma drug, wherein the at least one anti-glioblastoma drug is administered in a range of 0.01 to 100 mg/kg per body weight per day.

Preferred, the dose of the invention regarding chemotherapeutic agent, preferably the anti-glioblastoma drug is administered at least 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1.00, 1.05, 1.10, 1.15, 1.20, 1.25, 1.30, 1.35, 1.40, 1.45, 1.50, 2.00, 2.50, 3.00, 3.50, 4.00, 4.50, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5 or 15.0 mg/kg chemotherapeutic agent or anti-glioblastoma drug respectively per body weight per day.

Examples of such pharmaceutical compositions include, but are not limited to, solutions ready for injection, dry products ready to be dissolved or suspended in a pharmaceutically acceptable vehicle for injection, suspensions ready for injection, and emulsions.

Some suitable vehicles that can be used to provide pharmaceutical compositions for parenteral administration include, but are not limited to: Water for Injection USP; aqueous vehicles such as, but not limited to, sodium chloride injection, Ringer's injection, dextrose injection, dextrose and sodium chloride injection, and lactated Ringer's injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Suitable dosages of the active ingredients may be determined by a skilled medical practitioner. Actual dosage levels of the active ingredients may be varied so as to obtain an amount of the active ingredient which is effective to achieve the desired therapeutic response for a particular patient, composition, and mode of administration, without being toxic to the patient. Thus, the dosage is typically an effective or therapeutically effective dosage.

The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

Dosage regimens may be adjusted to provide the optimum desired response. For example, a single dose may be administered (e.g. a single dose daily), several divided doses may be administered over time or the dose may be proportionally reduced or increased as indicated by the exigencies of the therapeutic situation. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

In the compositions and products according to the invention, the active ingredients may each, for example, be present at a concentration of between 0.001 and 20% by weight, relative to the total weight of the composition or product, preferably between 0.01 and 10%, more preferably between 0.02 and 5% by weight, and more preferably still between 1 and 4% by weight. In a particular embodiment, each of the three active ingredients is present at a concentration of between 1 and 3% by weight.

In one currently preferred aspect, the artemisinin compound, in particular the artemisinin is formulated for administration at 50 to 500 mg (more preferably 100 to 300 mg such as about 200 mg) daily (based on a body weight of approximately 70 kg; dosages can be adjusted proportionally by body weight). Preferably, the artemisinin is formulated for oral or parenteral administration.

Preferably the artemisinin compound, in particular the artemisinin is administered for a period of 3 to 30 days, more preferably 10 to 20 days, such as about 14 days. This period may correspond to a cycle of treatment in an administration regime that comprises multiple such cycles of treatment (e.g. at least two, three, four, five, six, or more cycles, for example with such cycles continuing until the desired therapeutic results have been achieved). Each cycle of treatment may be separated by a break in the artemisinin compound, in particular artemisinin administration; such a break may, for example, allow for bone marrow recovery. The break in administration may comprise not administering the artemisinin compound, in particular the artemisinin for a period of 3 to 14 days, more preferably 5 to 10 days, such as about 7 days.

In one exemplary such aspect, the artemisinin compound, in particular the artemisinin is administered at about 200 mg daily for a cycle of treatment of two weeks, with each cycle being followed by a one week break.

In some embodiments, said pharmaceutical composition is used for prophylaxis and/or treatment of glioblastoma preferably in combination a radiotherapy, immunotherapy, electromagnetic field therapy and/or hyperthermia therapy.

In one preferred embodiment, said pharmaceutical composition is used for prophylaxis and/or treatment of glioblastoma wherein the glioblastoma is selected from subtypes proneural (PN), mesenchymal (MES), and classical (CL) glioblastoma.

### Description of Figures

**Figure 1****: Haploid screening in mouse stem cells delineates porphyrin biosynthesis as a prerequisite for artemisinin toxicity in mammalian cells. A** Schematic of haploid embryonic stem cell screens for compound target identification. **B** Porphyrin biogenesis pathway. Subcellular location (cytosol, mitochondria) and loss-of-function scores (italic) of major enzymes (bold, capitalised) and co-factors (normal, capitalised) of the screen (retroviral mutagenesis) are indicated. **C** GO-term analysis reveals porphyrin biogenesis as the major pathway targeted by artemisinin. **D** Competitive growth assay of artemisinin resistant single cell clones. Wild-type (mCherry+_ Cre) and knockout (GFP+) sister clones were derived from mutant (retrovirus - intron RE, darker shading, ToI2 transposon - intron T2, lighter shading) resistant colonies, treated with artemisinin, analysed with flow cytometry and sanger sequenced for insertion site mapping.
**Figure 2****: Modulation of porphyrin biosynthesis is sufficient to alter artemisinin toxicity in cells via ROS generation. A, B** Cell survival of Artemisinin treated mouse embryoni stem cells in combination with **A** the Ppox inhibitor acifluorfen or **B** 5-ALA (0.5mM). Alamar Blue staining was used to assess viability after 48 h of treatment. **C** Cell viability of artemisinin treated mouse breast cancer cells (4T1) in presence and absence of 5-ALA (0.5mM). Alamar Blue was used to determine cell survival after 48 h of treatment. **D** Survival of artemisinin treated primary human glioblastoma cells from cancer patients upon 5-ALA administration. Viability was assessed using CellTiter-Glo after 72h. Experiments were performed in triplicate. Values are mean ± SD. **E** ROS levels (DHE staining, PE 582/15 nm - MFI) and **F** cell survival of artemisinin (0.5µM), 5-ALA (0.25mM) or Ppox inhibitor (10µM) treated neuroblastoma cells (SHSY5Y). Fluorescence of DHE (ROS levels) and cell numbers (survival) were assessed by flow cytometry and automated cell counting. The experiments were performed two times, in triplicate each. Values indicate mean ± SD.
**Figure 3****: Cerebral tumor organoids (CNS-PNET) display increased sensitivity to artemisinin** and **5-ALA combination therapy. A** Human cerebral primitive neuroectodermal tumor organoids for compound profiling. **B** Schematic workflow representation and analysis of 5-ALA and artemisinin treated organoids. **C** Representative fluorescence and **D** brightfield images of DMSO (control), 5-ALA (0.0625mM), artemisinin (1µM), or 5-ALA and artemisinin (0.0625mM and 1µM) treated tumor organoids (CNS-PNET). Organoids were imaged on day 1, day 5 and day 7. Scale bar 500µm. **E** Image analysis and quantification of GFP-positive tumor area of treated organoids. Eight organoids per group were analysed on day 3 and normalized to day 1. Data is shown as box plots (25^{th}-75^{th} percentiles, median). **F** Flow cytometry analysis of dissociated cerebral tumor organoids. Relative percentages of GFP+ tumor cells of at least eight organoids per condition on day 5 normalised to control (DMSO) are shown. Box plots (25^{th}-75^{th} percentiles, median) of data are indicated. **G** ROS/ DHE staining and flow cytometry analysis of dissociated tumor organoids (PE, 582/15 nm). Mean fluorescence intensities (MFI) of DHE stained GFP+ tumor cells and GFP- control cells are indicated. n=2, values are mean ± SD. **H** Quantification of γH2AX+ cells in cerebral tumor organoids. 6 cryo-sections of 3 organoids each were stained for γH2AX, scanned and 25 regions of interest (ROI 2.500µm²) were analysed per condition. Raw numbers were normalised to organoid area (per ROI) and are shown as box plots (25th-75th percentiles, median).
**Figure 4****: Human glioblastoma-like neoplastic organoid models display increased sensitivity to artemisinin and 5-ALA combination therapy. A** Representative fluorescence (left panel) and brightfield (right panel) images of 5-ALA and artemisinin treated human cerebral tumor organoids. Organoids were monitored on day 1, day 5, day 8 and day 11. Scale bar 500µm. **B** Image analysis and quantification of treated organoids. Area of GFP-positive tumor cells on d11 was normalized to d1. Data is shown as box plots (25^{th}-75^{th} percentiles, median) n=4. **C** Representative brightfield images of 5-ALA and artemisinin treated patient derived glioblastoma spheroids (VBT92). Images were taken on day 3 of culture and treatment..
**Figure 5** **(related to** **Figure 1****): Artemisinin titration curves and an overview of the porphyrin biosynthesis pathway. A, B** Cell survival of artemisinin treated **A** mouse ESCs and **B** primary (MEF p3) fibroblasts, as well as mouse (B16F10, 4T1) and human (MDA-MB-231, Mcf7, Panel) cancer cells. Viability was assessed after 48h of treatment using Alamar Blue staining. **C** Porphyrin biosynthesis pathway component integrations sites from artemisinin screens. Genomic locations and targeted introns as well as exons of porphyrin biosynthesis genes (i.e. enzymes, bold), as well as retroviral (upper panel) or ToI2 transposon (lower panel) integrations sites (vertical bars) on the forward (black) and reverse (grey) strand are shown.
**Figure 6** **(related to** **Figure 2****): Effects of Artemisinin and 5-ALA combination treatment on survival, ROS production and mitochondrial membrane potential in cancer cell lines A** Cell survival of artemisinin treated mouse cancer cells (Mcf7 - breast cancer; B16F10 - melanoma) in presence and absence of 5-ALA (0.5mM). Alamar Blue was used to determine viability after 48h. **B, C** Cell survival of artemisinin treated primary human glioblastoma cells in presence or absence of 5-ALA. CellTiter-Glo was used to assess viability, 72h. Values are mean ± SD. **D, E** ROS/ DHE staining and flow cytometry analysis of piperlongumine or artemisinin treated **D** Jurkat and **E** HL-60 cells (48h). Values are mean ± SD. **F** ROS levels (DHE staining, PE 582/15 nm - MFI), **G** cell survival and **H** JC-1 levels of artemisinin (0.5µM), 5-ALA (0.25mM) or Ppox inhibitor (10µM) treated Jurkat cells, 48h. JC-1 negative cells have impaired mitochondrial membrane potential associated with apoptotic cell death. DHE fluorescence, relative cell numbers and percentage of JC-1 negative cells were assessed using high throughput flow cytometry and automated cell counting. All experiments were performed in triplicate and repeated once (JC-1) or twice (DHE, cell survival). Values are mean ± SD.
**Figure 7** **(related to** **Figure 3****): Cancer cell population in CNS-PNET tumor organoids displays high sensitivity to artemisinin and 5-ALA combination therapy. A** Cell survival of dissociated CNS-PNET tumor organoids. Quantification of GFP+ tumor cells and GFP- control cells of treated organoids normalised to control (DMSO) are shown. Data is shown as box plots (25^{th}-75^{th} percentiles, median). **B** Representative fluorescence (left panel) and brightfield (right panel) images of control (DMSO), 5-ALA (0.0625mM), artemisinin (1µM) or 5-ALA + artemisinin (0.0625mM and 1µM) treated cerebral tumor organoids. Scale bar 500µM. **C** Image quantification of GFP-positive tumor area on day 5 of treatment, as compared to day 1. Box plots (25^{th}-75^{th} percentiles, median) of data are shown. **D** Representative images of anti-Sox2, anti-GFP and DAPI stained sections of artemisinin and 5-ALA treated organoids. Scale bar 50µm.

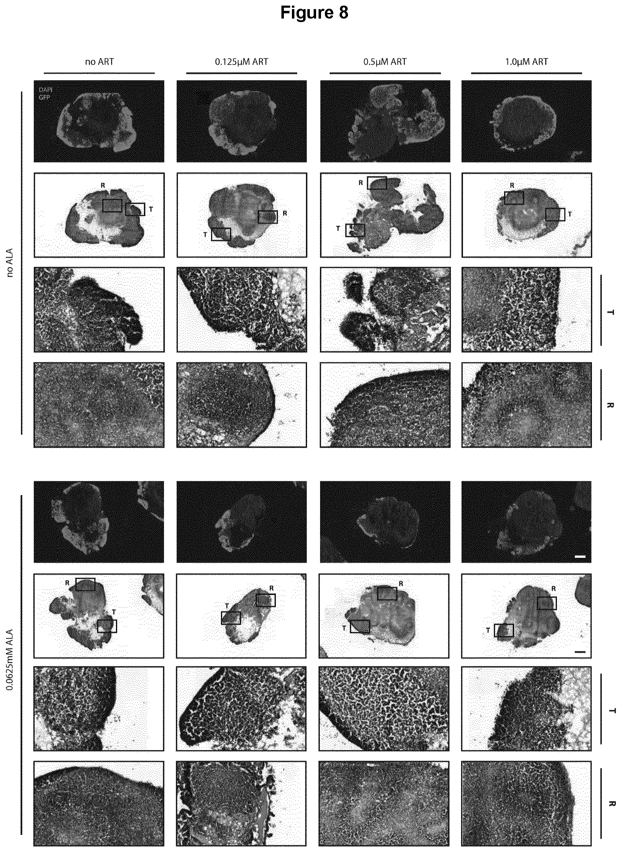

**Figure 8** **(related to** **Figure 3****): Staining of CNS-PNET tumor organoid sections following artemisinin and 5-ALA treatment.** Representative fluorescent (anti-GFP, DAPI) and H&E (haematoxylin & eosin stained) images of fixed cryo-sections of control and treated tumor organoids. Regions of rosette-like strucutres (R) or tumor tissue (T) are indicated and magnified (6.8x). Scale bar 500µM.
**Figure 9** **(related to** **Figure 3****): Analysis of CNS-PNET tumor organoids following artemisinin and 5-ALA treatment A** Representative FACS plots of ROS/ DHE stained dissociated tumor organoids. Flow cytometry analyses (PE, 582/15 nm) of GFP+ tumor cells and GFP- wild-type cells are shown. **B** Representative microscopy images of γH2AX, GFP and DAPI stained tumor organoid sections. Scale bar 50µM. **C** Representative images and analysis masks of γH2AX stained and scanned tumor organoid slides.

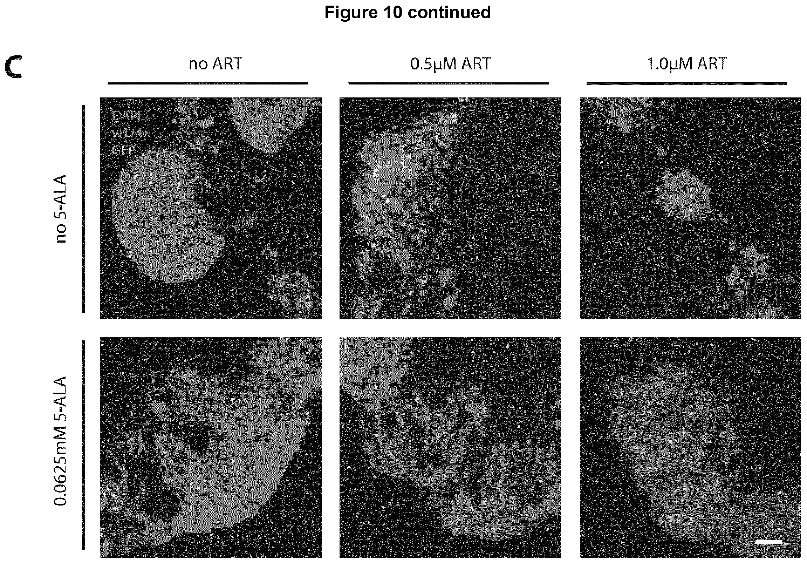

**Figure 10** **(related to** **Figure 3****): Continued analysis of CNS-PNET tumor organoids following artemisinin and 5-ALA treatment A** Quantification of Caspase 3 (Casp3) and **B** Ki67-positive cells in 5-ALA and artemisinin treated tumor organoids. Per condition and group, 3 organoids, 6 sections each, were stained with Caspase3 or Ki67, imaged using a high-magnification fluorescent scanner, and 25 regions of interest (ROI 2.500µm²) were chosen and analysed. The numbers of Casp3 or Ki67 positive cells were normalised to the analysed area (per ROI, GFP+ or GFP-) and are shown as box plots (median, 25th-75th percentiles). **C** Representative images of anti-Casp3, anti-GFP and DAPI stained sections. Scale bar 50µm.
**Figure 11** **(related to** **Figure 4****): Artemisinin and 5-ALA combination treatment of human glioblastoma-like neoplastic organoid models A** Fluorescence images of 5-ALA and artemisinin treated cerebral tumor organoids. Organoids were monitored on d1, d5, d8 and d11. Scale bar 500µm. **B** Image analysis and quantification of GFP-positive tumor area of treated organoids. Organoids were analysed on d8 and normalized to d1. Data is shown as box plots (25^{th}-75^{th} percentiles, median).
**Figure 12****: Artemisinsin (ART) and its derivatives Dihydroartemisinin (DHA) and Artesunate (ARS) exert synergistic antiproliferative effects on glioblastoma cell lines and mouse embryonic stem cells (ESC) when combined with 5-Aminolevulinic acid (5-ALA).**
   Shown are 6-day titration curves of artemisinin compounds and 5-ALA. **A-R** Cell survival of 5-ALA treated glioblastoma lines (as indicated) and ESC in combination with the indicated **A, D, G, J** , **M P** ART doses, or **B, E, H, K, N, Q** DHA doses, or **C, F, I, L, O, R** ARS doses compared to untreated controls. Values are Mean ±SEM from ≥ 3 Independent experiments. Dotted lines mark 100% survival.
**Figure 13****: ART or DHA combined with 5-ALA increases the anti proliferative effect of Temozolomide (TMZ) treatment in glioblastoma cell lines and ESC.**
   **A-L** 5-ALA and TMZ treated glioblastoma lines (as indicated) and ESC in combination with the indicated **A, C, E, G, I, K** ART doses or **B, D, F, H, J, L** DHA doses compared to untreated controls. Values are Mean +SEM from ≥ 5 Independent experiments. Dotted lines mark 100% survival and the survival upon TMZ treatment only.
**Figure 14****: ARS combined with 5-ALA or Methyl-5-ALA increases the antiproliferative effect of Temozolomide (TMZ) treatment in glioblastoma cell lines and ESC. 5-ALA is more potent than Methyl-5-ALA in this setup.**
   **A-F** 5-ALA and TMZ treated glioblastoma lines (as indicated) and ESC in combination with the indicated ARS doses compared to untreated controls. Values are Mean +SEM from ≥ 3 Independent experiments. Dotted lines mark 100% survival and the survival upon TMZ treatment only.
**Figure 15****: ART combined with 5-ALA increases the antiproliferative effect of Lomustine (CCNU) treatment in glioblastoma cell lines and ESC.**
   **A-F** 5-ALA and CCNU treated glioblastoma lines (as indicated) and ESC in combination with ART doses compared to untreated controls. Values are Mean +SEM from ≥ 4 Independent experiments. Dotted lines mark 100% survival and the survival with CCNU treatment only.

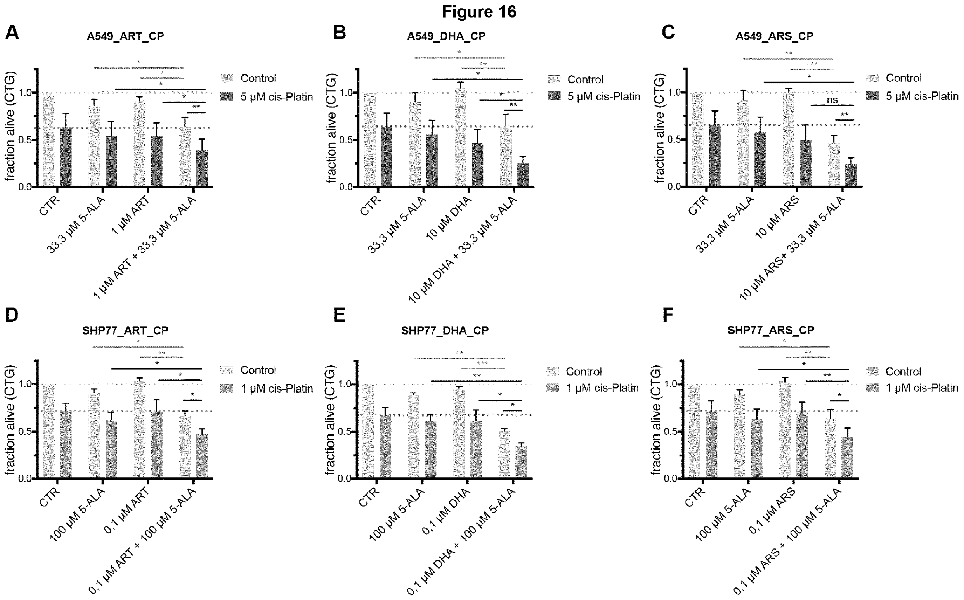

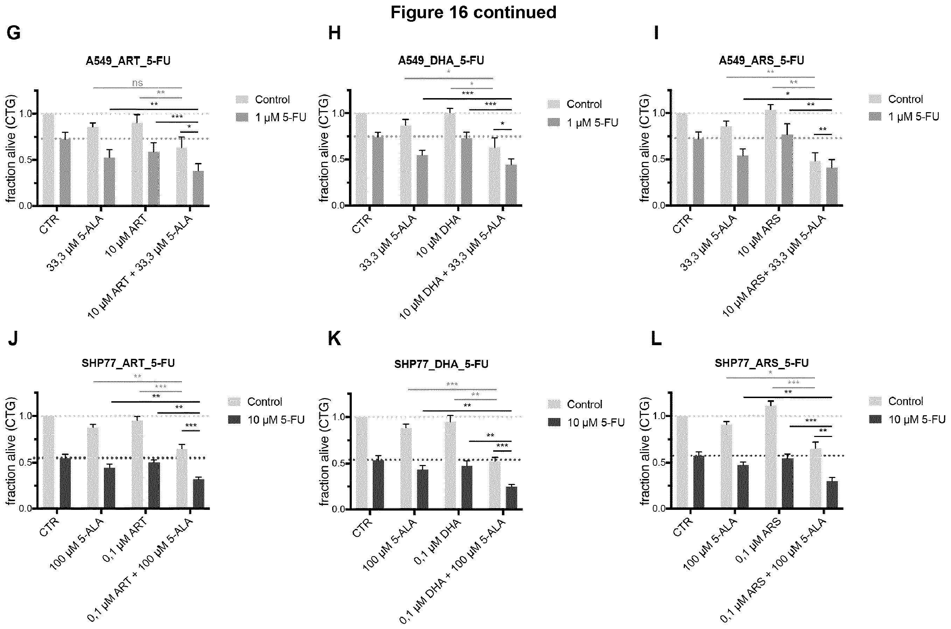

**Figure 16****: ART, DHA, or ARS combined with 5-ALA increases the anti proliferative effect of cisplatin (CP) and 5-Fluorouracil (5-FU) treatment in two lung cancer cell lines.**
   **A-L** Cell survival of 5-ALA treated lung cancer lines (as indicated) in combination with the indicated **A, D, G, J** ART doses, or **B, E, H, K,** DHA doses, or **C, F, I, L,** ARS doses and either cisplatin (**A-F**) or 5-FU (**G-L**) conditioning compared to untreated controls. Values are Mean +SEM from ≥ 4 Independent experiments. Dotted lines mark 100% survival and the survival upon chemotherapeutic (cisplatin or 5-FU) treatment only.

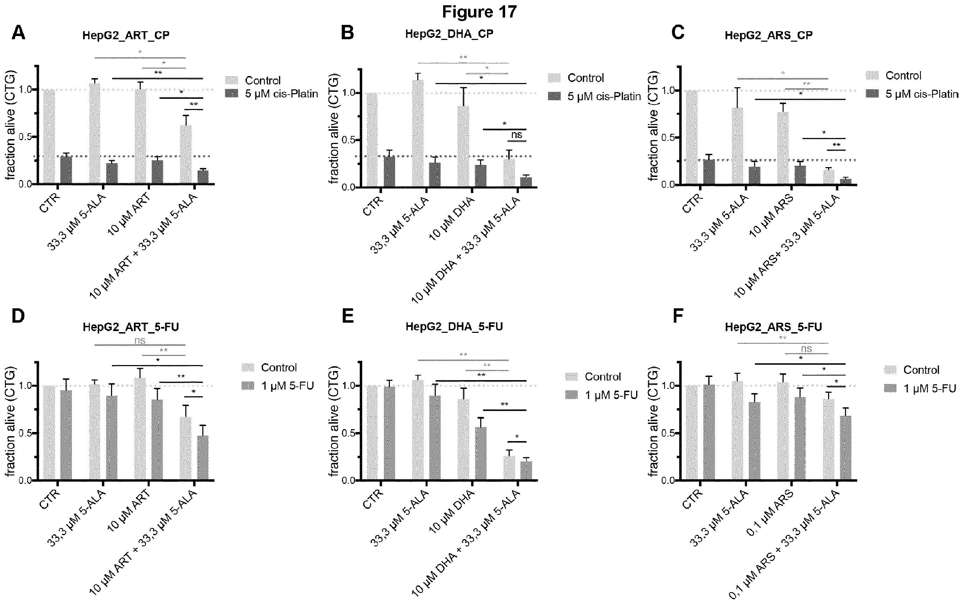

**Figure 17****: ART, DHA, or ARS combined with 5-ALA increases the anti proliferative effect of cisplatin (CP) and 5-Fluorouracil (5-FU) treatment in HepG2 cells (liver cancer).**
   **A-F** Cell survival of 5-ALA treated HepG2 cells in combination with the indicated **A, D,** ART doses, or **B, E,** DHA doses, or **C, F,** ARS doses and either cisplatin (**A-C**) or 5-FU (**D-F**) conditioning compared to untreated controls. Values are Mean +SEM from ≥ 4 Independent experiments. Dotted lines mark 100% survival and the survival upon chemotherapeutic (cisplatin or 5-FU) treatment only.

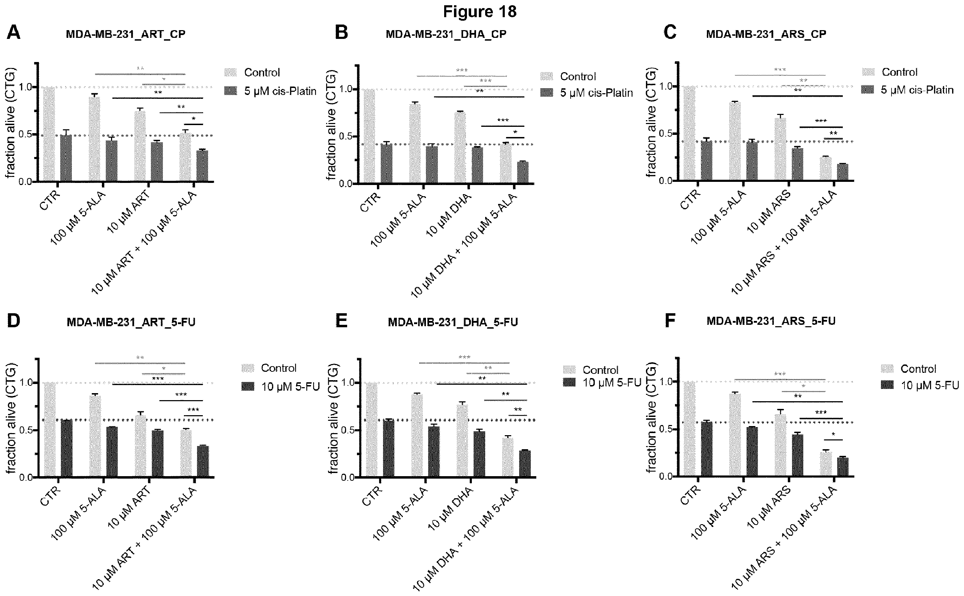

**Figure 18****: ART, DHA, or ARS combined with 5-ALA increases the anti proliferative effect of cisplatin and 5-fluorouracil (5-FU) treatment in MD-MBA-231 cells (breast cancer).**
   **A-F** Cell survival of 5-ALA treated MD-MBA-231 cells in combination with the indicated **A, D,** ART doses, or **B, E,** DHA doses, or **C, F,** ARS doses and either cisplatin (**A-C**) or 5-FU (**D-F**) conditioning compared to untreated controls. Values are Mean +SEM from ≥ 4 Independent experiments. Dotted lines mark 100% survival and the survival upon chemotherapeutic (cisplatin or 5-FU) treatment only.

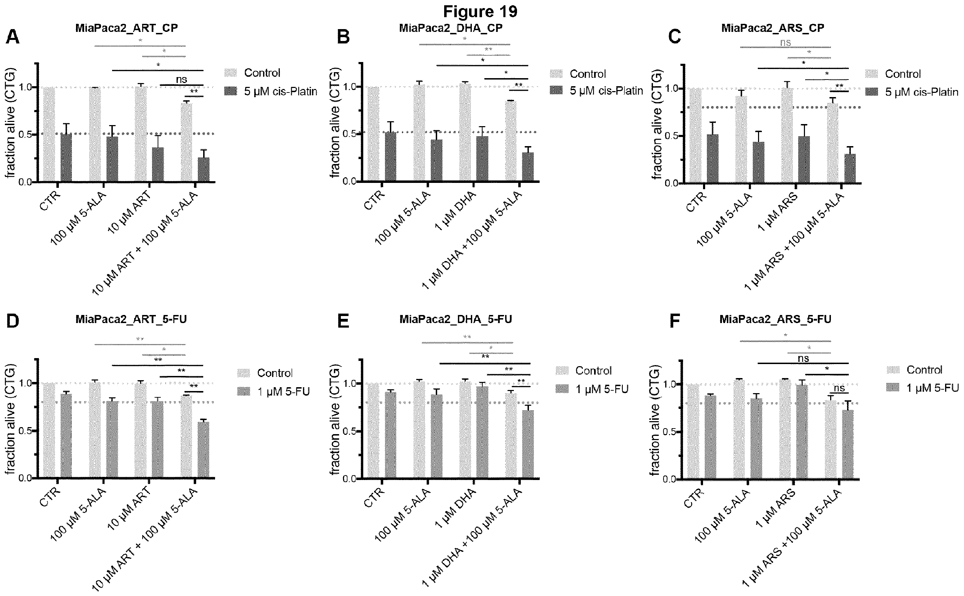

**Figure 19****: ART, DHA, or ARS combined with 5-ALA increases the antiproliferative effect of cisplatin and 5-fluorouracil (5-FU) treatment in MiaPaca-2 cells (pancreas cancer).**
   **A-F** Cell survival of 5-ALA treated MiaPaca-2 cells in combination with the indicated **A, D,** ART doses, or **B, E,** DHA doses, or **C, F,** ARS doses and either cisplatin (**A-C**) or 5-FU (**D-F**) conditioning compared to untreated controls. Values are Mean +SEM from ≥ 4 Independent experiments. Dotted lines mark 100% survival and the survival upon chemotherapeutic (cisplatin or 5-FU) treatment only.

### Examples

### Materials and Methods

### Mammalian tissue culture

Mouse embryonic stem cell clones (clone AN3-12) {Elling:2011gla} were cultured in DMEM supplemented with 10% fetal bovine serum (FCS), penicillin-streptomycin, non-essential amino acids, sodium pyruvate (1 mM), I-glutamine (2 mM), β-mercaptoethanol (0.1 mM), and LIF (20 µg ml-1). SH-SY5Y cells were cultured in DMEM/F12 1:1 supplemented with 10% FCS (fetal calf serum), penicillin-streptomycin, and L-glutamine. 4T1 cells were cultured in IMDM supplemented with 10% fetal calf serum (FCS), penicillin-streptomycin and L-glutamine. MEFs, Mcf7, MDA-MB-231, Panc1, LN229, A549, MiaPaca-2, B16F10 and PlatE cells were cultured in DMEM supplemented with 10% FCS, penicillin-streptomycin and L-glutamine. HepG2-, T98G-, and U87MG cells were cultured in EMEM, and SHP77-, VBT92-, and VBT281 cells were cultured in RPMI, each supplemented with 10% FCS, penicillin-streptomycin and L-glutamine. All cells were cultured at 37°C, on f 20%O₂ and 5%CO₂.

### Cell lines

Mouse AN3-12 ESC lines were generated in our laboratory and characterized and authenticated as previously described {Elling, U. et al. Forward and reverse genetics through derivation of haploid mouse embryonic stem cells. Cell stem cell 9, 563-574 (2011)}. Haploid murine ESCs were used for insertional mutagenesis and derivation of gene trap knockout cell lines. SH-SY5Y were obtained directly from the supplier (Sigma Aldrich) and used for growth assays and cellular stainings. Jurkat cells as used in *in vitro* viability and DHE assays were obtained from an in-house source and are functionally described elsewhere {Reikerstorfer, A., Holz, H., Stunnenberg, H. G. & Busslinger, M. Low affinity binding of interleukin-1 beta and intracellular signaling via NF-kappa B identify Fit-1 as a distant member of the interleukin-1 receptor family. The Journal of biological chemistry 270, 17645-17648 (1995)}. Mcf7, MDA-MB-231, 4T1, Panc1 and B16F10 cancer cell lines were obtained in house. MEFs were generated and obtained in our laboratory. PlatE cells were used for recombinant retrovirus and lentivirus production as previously described {Taubenschmid, J. et al. A vital sugar code for ricin toxicity. Cell Research 27, 1351-1364 (2017). Stadlmann, J. et al. Comparative glycoproteomics of stem cells identifies new players in ricin toxicity. Nature 549, 538-542 (2017)}. All cell lines were tested negative for mycoplasma. No cell line listed by ICLAC was used.

### Competitive growth assays

Haploid ESCs harbouring gene traps in nomic introns were seeded at low density in normal ESC growth medium and infected for 12h with two viruses, one encoding mCherry plus Cre recombinase and the other coding for GFP, both together with puromycin (Invivogen, ant-pr-1). Infected cells were selected (final concentration of puromycin, 1 µg/ml) after 24h and expanded. Ratios of GFP- to mCherry/Cre-expressing cells in the presence and absence of artemisinin were determined using high-throughput flow cytometry (BD LSRFortessa HTS Cell Analyzer).

### Compound profiling in cell lines

For dosage responses, cells were seeded in 96-wells (25.000/ 96-well, in triplicate - where indicated) and subjected to compounds for 48h. Cell viability was assessed using automated cell counting (high-throughput flow cytometry), Alamar Blue staining (Invitrogen, DAL1100) or CellTiter-Glo Luminescent Assay (Promega, G7570, according to the manufacturer's protocol) respectively.

Cell survival assays (Figures 12-19) were performed in 96-well plates in technical duplicates. Treatments started 24 hours post cell plating and lasted for 6-days prior to cell viability assessment with the Cell Titer Glo 2.0 Luminescent assay (Promega, G9242).

### Dihydroethidium (DHE) staining

Treated cells were collected, washed with 1x HBSS (without Ca2+ and Mg2+), incubated with 1mM DHE (Dihydroethidium (Hydroethidine), Invitrogen, D11347) in 1x HBSS for 45min at 37°C, washed twice and counterstained with DAPI or a viability dye (eBioscience^{™} Fixable Viability Dye eFluor^{™} 780, 65-0865-18) for 10min on ice. Cells were then collected, strained and analysed using flow cytometry for DHE in the red fluorescent spectrum (PE channel).

### JC-1 staining

Cells were collected, washed with 1xPBS, incubated with 2µM JC-1 in 1PBS (MitoProbe JC-1 Assay Kit-1, Invitrogen, M34152) for 35min at 37°C, washed twice, and analysed using flow cytometry in the red (PE channel) and green (FITC channel) fluorescence spectrum.

### Cerebral organoid formation

Cerebral organoids were generated as described before { Lancaster, M. A. et al. Cerebral organoids model human brain development and microcephaly. Nature 501, 373-379 (2013)}. Human embryonic stem cells (feeder-free H9, WiCell) were transferred into low-attachment 96-well-plates (Corning) in a density of 9.000 cells per well and incubated in human stem cell medium. After 6 days, the medium was changed to neural induction media, containing Dulbecco's modified eagle medium DMEM/F12, N2 supplement (Invitrogen), Glutamax (Invitrogen), minimum essential media-nonessential amino acids (MEM-NEAA) and 1µg/ml heparin (Sigma), to promote growth of ectodermal tissue. On day 11, embryonic bodies (EBs) were embedded in droplets of Matrigel and transferred to differentiation medium, containing DMEM/F12: Neurobasal 1:1, N2 supplement (Invitrogen), B27 supplement (without vitamin A) (Invitrogen), 50µM 2-mercaptoethanol, 1:4,000 insulin (Sigma), Glutamax (Invitrogen), penicillin-streptomycin, MEM-NEAA onto 10cm plates. 5 days later, organoids were transferred to an orbital shaker and maintained in differentiation media containing vitamin A (in B27 supplement).

### Cerebral tumor organoid formation + Nucleofection

Tumor initiation was induced by either oncogene amplification, using a Sleeping Beauty (SB) transposase, or mutation of tumor suppressor genes, using the CRISPR-Cas9 system in day 10 old embryoid bodies (EBs). Plasmids carrying the transposase as well as GFP and the desired oncogenes or expressing Cas9 nuclease were introduced by electroporation. In brief, a mixture of 1µg DNA and 100µl nucleofector solution was added to 10 EBs and transferred to a nucleofection cuvette. The Lonza Nucleofector 2b and the A-023 program were used for electroporation/ nucleofection. Thereafter, EBs were transferred into 10cm dishes containing differentiation media containing vitamin A and embedded into Matrigel 24 hours later. Two different types of tumors were initiated {Bian:2018gs}: Central nervous system primitive neuroectodermal tumors (CNS-PNET) were induced by the overexpression of Myc. Glioblastoma-like tumor group 2 (GBM-2) was caused by mutagenesis of the tumor suppressor genes p53, NF1 and PTEN. All plasmids were designed by Shan Bian {Bian, S. et al. Genetically engineered cerebral organoids model brain tumor formation. Nat. Methods 15, 631-639 (2018)}.

### Compound profiling in cerebral tumor organoids

Cerebral tumor organoids were treated with different compounds and the survival and growth of transformed as well as untransformed neurons was monitored. Fluorescence labeling of tumor cells allowed clear distinguishability of transformed cells as compared to non-labeled throughout the experiment using brightfield microscopy, as well as fluorescence imaging. The transformed tumor tissue and untransformed neurons within the same organoid was monitored using fluorescence imaging, as well as brightfield microscopy throughout the experiment. At the endpoint of the treatment (d5 or d7 respectively), the number of GFP-positive cells was assessed by flow cytometry.

### Flow cytometry analysis of cerebral tumor organoids

Cerebral (tumor) organoids were enzymatically and mechanically dissociated using 1x trypsin, 35-45min incubation at 37°C, and gentle shaking. Organoids were singularised by careful resuspension, addition of differentiation medium and straining (Falcon Round-Bottom Tubes with Cell Strainer Cap, 5 mL, 35 µm nylon mesh cell strainer snap cap). Single cells in suspension were counterstained with DAPI or a viability dye (eBioscience^{™} Fixable Viability Dye eFluor^{™} 780, 65-0865-18), and analysed using flow cytometry (BD LSRFortessa HTS Cell Analyzer). The amounts of GFP-positive cells were analyzed.

### Imaging of cerebral tumor organoids

5-ALA or Artemisinin treated cerebral organoids were imaged at the indicated time points using the Axio Vert.A1 Inverted Microscope system (Zeiss Objective EC Plan-Neofluar 2.5x/0.085 Pol M27, 0.5 camera adapter). Brightfield and green fluorescence images were taken from the same area. Additionally, for image analyses, the Celldiscoverer 7 (Zeiss), a fully integrated high-end automated live cell imaging system, was used.

### Immunohistochemistry staining

Cerebral organoids were fixed in 4% PFA (room temperature, 1h), incubated with 30% sucrose (4°C, o/n), OCT (Tissue-tek OCT Compound, SANOVA PHARMA GESMBH, 4583) embedded and 20µm sections were cryostat cut (-12/-14°C). For staining, sections were blocked and permeabilized for 1h on RT in 0.25% Triton X-100, 4% donkey serum in PBS, stained with primary antibodies diluted in in 0.1% Triton X-100, 4% donkey serum in PBS at RTovernight, incubated with secondary antibodies diluted in in 0.1% Triton X-100, 4% donkey serum in PBS for 2h at RT and counterstained with DAPI (4',6-Diamidino-2-Phenylindole, Dilactate, Invitrogen, D3571) at RT for 20min. Fluorescence Mounting Medium (Dako, S302380) was used for mounting the sample slides. The organoids were imaged on an LSM780 Axio Imager (point laser scanning confocal microscope, GaAsP (Gallium Arsenide) detectors with QE of 45% and up to 2x SNR) with a standard filter set (CH1: 371-735, CH2: 479-735, CH3 Quasar (GaAsP): 416-690) through a 20x/0.8 plan-Apochromat objective (Carl Zeiss) using laser illumination (Laser Diode 405 - 25mW, Argon 458, 488, 514 - 30mW, DPSS 561 - 15mW, HeNe 633 - 5mW).

### Statistics and reproducibility

All values in Figures 1-11 are given as means ± SD, unless stated otherwise. All experiments were reproduced at two to seven independent times, with similar results.

GraphPad Prism was used to generate figures and perform statistical analyses (GraphPad Software). An a *priori* sample size estimation was not performed. The experiments were not randomized. The investigators were not blinded to allocation during experiments and outcome assessment. Data were analysed by using the unpaired two-tailed Student's t-test, as indicated. P < 0.05 was accepted as statistically significant. Box and whisker plots depict the median and ranges from the first to the third quartile.

For data represented in Figures 12-19, all values are given +SEM. Data were analyzed by using the paired two-tailed Student's t-test in line with the experimental setup. Significance is indicated as follows: * p<0.05, **p<0.01, ***p<0.001.

## Claims

1. A pharmaceutical composition comprising
a) an artemisinin compound (**1**) selected from 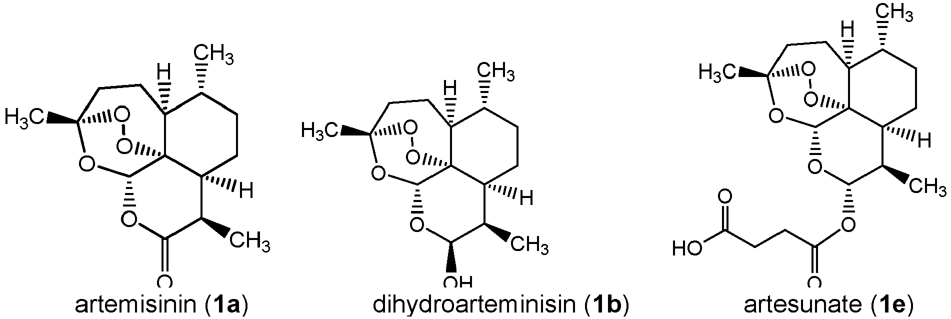 or a pharmaceutically acceptable salt, cocrystal, or solvate thereof;
b) 5-aminolevulinic acid (**2**), methyl-5-aminolevulinic acid (**2b**), or a pharmaceutically acceptable salt or solvate thereof; and
c) at least one chemotherapeutic agent selected from the group consisting of temozolomide, lomustine, cisplatin, and 5-fluorouracil.

2. The pharmaceutical composition according to claim 1, wherein a molar ratio of the artemisinin compound (**1**) and the 5-aminolevulinic acid (**2**) or the methyl-5-aminolevulinic acid (**2b**) is in a range of 1:5 to 1:5000.

3. The pharmaceutical composition according to any one of the claims 1 - 2, wherein the pharmaceutical composition further comprises a pharmaceutically acceptable carrier, excipient and/or diluent.

4. A pharmaceutical composition according to any one of the claims 1 - 3 for use in the prophylaxis and/or treatment of hematopoietic cancers, brain cancer, pancreatic cancer, liver cancer, breast cancer, and lung cancer and preferably brain cancer.

5. The pharmaceutical composition for use according to claim 4, wherein the pharmaceutical composition is in a form of a tablet, capsule, syrup, solution, suspension, emulsion, or gel.

6. The pharmaceutical composition for use according to any one of the claims 4-5, wherein the pharmaceutical composition is administered by oral, intratecal, intravenous, subcutaneous, parenteral application or by inhalation.

7. The pharmaceutical composition for use according to any one of the claims 4-6, wherein the artemisinin compound (1) is administered in a range of 0.01 to 100 mg/kg per body weight per day and the 5-aminolevulinic acid or the methyl-5-aminolevulinic acid is administered in a range of 0.01 to 200 mg/kg per body weight per day.

8. The pharmaceutical composition for use according to any one of the claims 4-7, wherein the at least one chemotherapeutic agent is administered in a range of 0.01 to 100 mg/kg per body weight per day.

9. The pharmaceutical composition for use according to any one of the claims 4 - 8, wherein the brain cancer is selected from subtypes proneural (PN), mesenchymal (MES), and classical (CL) glioblastoma.

10. The pharmaceutical composition for use according to any one of the claims 4 - 9, wherein the prophylaxis and/or treatment of hematopoietic cancers, brain cancer, pancreatic cancer, liver cancer, breast cancer, and lung cancer and preferably brain cancer is performed in combination with a radiotherapy, immunotherapy, electromagnetic field therapy, hyperthermia therapy, chemotherapy, cancer immunotherapy and/or any other small molecule based therapy.

## Patentansprüche

1. Eine pharmazeutische Zusammensetzung umfassend
a) eine Artemisinin-Verbindun (**1**) ausgewählt aus 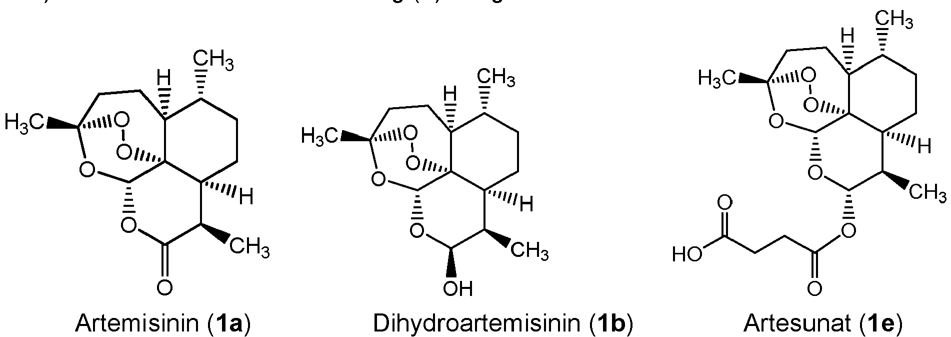 oder ein pharmazeutisch verträgliches Salz, Co-Kristall oder Solvat davon;
b) 5-Aminolävulinsäure (**2**), Methyl-5-aminolävulinsäure (**2b**), oder ein pharmazeutisch verträgliches Salz oder Solvat davon; und
c) mindestens ein chemotherapeutisches Mittel ausgewählt aus der Gruppe bestehend aus Temozolomid, Lomustin, Cisplatin und 5-Fluoruracil.

2. Die pharmazeutische Zusammensetzung gemäß Anspruch 1, wobei ein molares Verhältnis der Artemisinin-Verbindung (**1**) und der 5-Aminolävulinsäure (**2**) oder der Methyl-5-aminolävulinsäure (**2b**) in einem Bereich von 1:5 bis 1:5000 liegt.

3. Die pharmazeutische Zusammensetzung gemäß einem der Ansprüche 1 - 2, wobei die pharmazeutische Zusammensetzung weiter einen pharmazeutisch verträglichen Träger, Hilfsstoff und/oder ein Verdünnungsmittel umfasst.

4. Eine pharmazeutische Zusammensetzung gemäß einem der Ansprüche 1 - 3 zur Verwendung bei der Prophylaxe und/oder Behandlung von hämatopoetischen Krebserkrankungen, Hirnkrebs, Bauchspeicheldrüsenkrebs, Leberkrebs, Brustkrebs und Lungenkrebs und bevorzugt Hirnkrebs.

5. Die pharmazeutische Zusammensetzung zur Verwendung gemäß Anspruch 4, wobei die pharmazeutische Zusammensetzung in Form einer Tablette, Kapsel, eines Sirups, einer Lösung, Suspension, Emulsion oder eines Gel vorliegt.

6. Die pharmazeutische Zusammensetzung zur Verwendung gemäß einem der Ansprüche 4 - 5, wobei die pharmazeutische Zusammensetzung durch orale, intrathekale, intravenöse, subkutane, parenterale Anwendung oder durch Inhalation verabreicht wird.

7. Die pharmazeutische Zusammensetzung zur Verwendung gemäß einem der Ansprüche 4 - 6, wobei die Artemisinin-Verbindung (**1**) in einem Bereich von 0,01 bis 100 mg/kg pro Körpergewicht pro Tag verabreicht wird und die 5-Aminolävulinsäure oder die Methyl-5-aminolävulinsäure in einem Bereich von 0,01 bis 200 mg/kg pro Körpergewicht pro Tag verabreicht wird.

8. Die pharmazeutische Zusammensetzung zur Verwendung gemäß einem der Ansprüche 4 - 7, wobei das mindestens eine chemotherapeutische Mittel in einem Bereich von 0,01 bis 100 mg/kg pro Körpergewicht pro Tag verabreicht wird.

9. Die pharmazeutische Zusammensetzung zur Verwendung gemäß einem der Ansprüche 4 - 8, wobei der Hirnkrebs ausgewählt wird aus den Subtypen proneurales (PN), mesenchymales (MES) und klassisches (CL) Glioblastom.

10. Die pharmazeutische Zusammensetzung zur Verwendung gemäß einem der Ansprüche 4 - 9, wobei die Prophylaxe und/oder Behandlung von hämatopoetischen Krebserkrankungen, Hirnkrebs, Bauchspeicheldrüsenkrebs, Leberkrebs, Brustkrebs und Lungenkrebs und bevorzugt Hirnkrebs in Kombination mit einer Strahlentherapie, Immuntherapie, Elektromagnetfeldtherapie, Hyperthermietherapie, Chemotherapie, Krebsimmuntherapie und/oder einer anderen auf niedermolekularen Verbindungen basierten Therapie durchgeführt wird.

## Revendications

1. Une composition pharmaceutique comprenant
a) un composé d'artémisinine (**1**) choisi à partir de 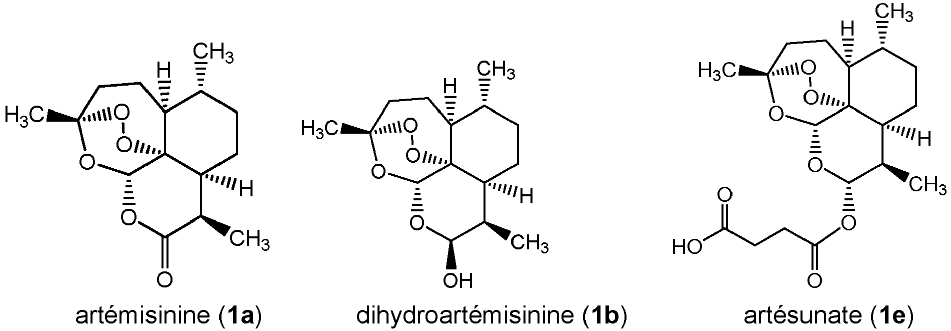 ou d'un sel, d'un cocristal ou d'un solvate des produits susmentionnés de formule acceptable dans le domaine pharmaceutique ;
b) de l'acide 5-aminolévulinique (**2**), de l'acide méthyl-5-aminolévulinique (**2b**), ou l'un de ses sels ou solvates acceptables dans le domaine pharmaceutique ;
c) au moins un agent chimiothérapeutique choisi dans le groupe constitué par le témozolomide, la lomustine, le cisplatine et le 5-fluorouracile.

2. La composition pharmaceutique selon la revendication 1, où le rapport molaire entre le composé d'artémisinine (**1**) et l'acide 5-aminolévulinique (**2**) ou l'acide méthyl-5-aminolévulinique (**2b**) est compris entre 1:5 et 1:5000.

3. La composition pharmaceutique conforme à l'une des revendications 1 à 2, où ladite composition pharmaceutique comprend également au moins un vecteur, un excipient et/ou un diluant acceptable dans le domaine pharmaceutique.

4. Une composition pharmaceutique conforme à l'une des revendications 1 à 3 pour utilisation dans la prophylaxie et/ou le traitement des cancers hématopoïétiques, du cancer du cerveau, du cancer du pancréas, du cancer du foie, du cancer du sein et du cancer du poumon, et de préférence du cancer du cerveau.

5. Une composition pharmaceutique destinée à une utilisation conforme à la revendication 4, où ladite composition pharmaceutique se présente sous la forme d'un comprimé, d'une capsule, d'un sirop, d'une solution, d'une suspension, d'une émulsion ou d'un gel.

6. Une composition pharmaceutique destinée à une utilisation conforme à l'une des revendications 4 à 5, où ladite composition pharmaceutique est administrée par voie orale, intrathécale, intraveineuse, sous-cutanée, parentérale ou par inhalation.

7. Une composition pharmaceutique destinée à une utilisation conforme à l'une des revendications 4 à 6, où l'artémisinine (**1**) est administrée à raison de 0,01 à 100 mg/kg de poids corporel par jour et l'acide 5-aminolévulinique ou l'acide méthyl-5-aminolévulinique est administré à raison de 0,01 à 200 mg/kg de poids corporel par jour.

8. Une composition pharmaceutique destinée à une utilisation conforme à l'une des revendications 4 à 7, où au moins un agent chimiothérapeutique est administré dans une fourchette de 0,01 à 100 mg/kg de poids corporel par jour.

9. Une composition pharmaceutique destinée à une utilisation conforme à l'une des revendications 4 à 8, où le cancer du cerveau est choisi parmi les sous-types de glioblastome proneural (PN), mésenchymateux (MES) et classique (CL).

10. Une composition pharmaceutique destinée à une utilisation conforme à l'une des revendications 4 à 9, où la prophylaxie et/ou le traitement des cancers hématopoïétiques, du cancer du cerveau, du cancer du pancréas, du cancer du foie, du cancer du sein et du cancer du poumon, et de préférence du cancer du cerveau, sont effectués en combinaison avec une radiothérapie, une immunothérapie, une thérapie par champ électromagnétique, une thérapie par hyperthermie, une chimiothérapie, une immunothérapie anticancéreuse et/ou toute autre thérapie à base de petites molécules.
